(19) Europäisches Patentamt / European Patent Office / Office européen des brevets

(11) **EP 4 421 063 A1**

(12) **EUROPEAN PATENT APPLICATION**
published in accordance with Art. 153(4) EPC


(43) Date of publication:
**28.08.2024 Bulletin 2024/35**

(21) Application number: **22883592.2**

(22) Date of filing: **19.10.2022**

(51) International Patent Classification (IPC):
***C07C 68/06*** *(2020.01)* ***C07C 68/08*** *(2006.01)*
***C07C 69/96*** *(2006.01)*

(52) Cooperative Patent Classification (CPC):
(C-Sets available)
**C07C 68/06; C07C 68/08;** Y02P 20/10 (Cont.)

(86) International application number:
**PCT/JP2022/038887**

(87) International publication number:
**WO 2023/068288 (27.04.2023 Gazette 2023/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.10.2021 JP 2021172658**



(71) Applicant: **Asahi Kasei Kabushiki Kaisha**
**Tokyo 1000006 (JP)**

(72) Inventor: **AKATSUKA, Kenji**
**Tokyo 100-0006 (JP)**

(74) Representative: **Strehl Schübel-Hopf & Partner**
**Maximilianstrasse 54**
**80538 München (DE)**


(54) **METHOD FOR PRODUCING DIPHENYL CARBONATE**


(57) Provided is a method for producing diphenyl carbonate, comprising a first purification step of continuously introducing a reaction mixture containing diphenyl carbonate obtained by specific steps to a high-boiling substance separation column A, and continuously separating by distillation the mixture into a column top component ($A_T$) containing diphenyl carbonate and a column bottom component ($A_B$) containing a catalyst; a specific second purification step; and two specific circulation steps, wherein a mass concentration ratio (DPC/HB) of diphenyl carbonate to a high-boiling substance in the column bottom component ($A_B$) of the high-boiling substance separation column A is 0.1 to 1.0, and a mass concentration ratio (DPC/catalyst) of the diphenyl carbonate to the catalyst is 1.0 to 10.0.


[Figure 5]

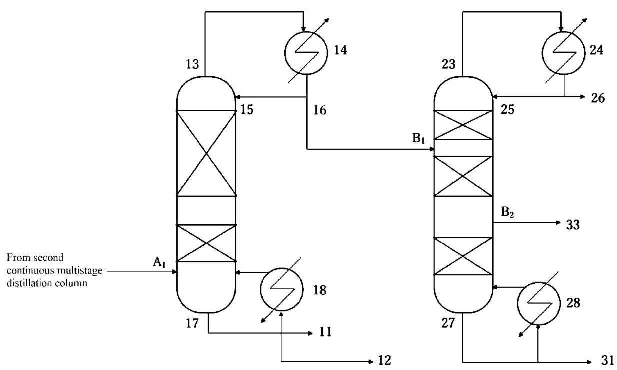


EP 4 421 063 A1

(52) Cooperative Patent Classification (CPC): (Cont.)

C-Sets
**C07C 68/06, C07C 69/96;**
**C07C 68/08, C07C 69/96**

## Description

### Technical Field

The present invention relates to a method for producing diphenyl carbonate.

### Background Art

Reaction mixtures obtained through transesterification reaction with dialkyl carbonate and phenol as raw materials in the presence of a homogeneous catalyst usually contain various reaction by-products. Particularly, diphenyl carbonate without reduction to (below) a sufficient level in the amount of high-boiling by-products, such as phenyl salicylate, xanthone, phenyl methoxybenzoate, or 1-phenoxycarbonyl-2-phenoxycarboxy-phenylene, having a higher boiling point than that of the diphenyl carbonate of interest causes stain or reduction in physical properties if used as a raw material for polycarbonate in a transesterification method. It is thus preferred to minimize the amount of these impurities. As a method for solving these problems, for example, Patent Document 1 proposes a method for producing high-purity diphenyl carbonate necessary for the production of high-quality and high-performance aromatic polycarbonate from cyclic carbonate and phenol, comprising the steps of: (I) producing dialkyl carbonate and diols using a reactive distillation column having a specific structure; (II) producing diphenyl carbonate using two reactive distillation columns having a specific structure; and (III) obtaining high-purity diphenyl carbonate therefrom using a high-boiling substance separation column A and a diphenyl carbonate purifying column B, whereby high-purity diphenyl carbonate is stably produced in an industrially large amount (e.g., 1 ton or more per hour) for a long period (e.g., 1000 hours or longer, preferably 3000 hours or longer, more preferably 5000 hours or longer).

### List of Prior Art Documents

### Patent Document

Patent Document 1: International Publication No. 2007/072705

### Summary of Invention

### Problems to be Solved by Invention

In the method described in Patent Document 1, alkyl aryl carbonate (e.g., methyl phenyl carbonate (MPC)) and an aliphatic alcohol (e.g., methanol (MeOH)) are formed through the reaction of alkyl carbonate (e.g., dimethyl carbonate (DMC)) with phenol (PhOH) in the presence of a catalyst in the first continuous multistage distillation column, and diaryl carbonate (e.g., diphenyl carbonate (DPC)) and dialkyl carbonate (e.g., dimethyl carbonate(DMC)) are formed through disproportionation reaction of alkyl aryl carbonate (e.g., methyl phenyl carbonate (MPC)) in the second continuous multistage distillation column. These reactions are represented by the following reaction formulas:

(First continuous multistage distillation column)

$$\text{DMC} + \text{PhOH} \Leftrightarrow \text{MPC} + \text{MeOH}$$

(Second continuous multistage distillation column)

$$2\text{MPC} \Leftrightarrow \text{DPC} + \text{DMC}$$

After the disproportionation reaction, a high-boiling reaction mixture of the second continuous multistage distillation column containing diaryl carbonate (e.g., diphenyl carbonate (DPC)) is distilled by a high-boiling substance separation column A and a diphenyl carbonate purifying column B. In this context, a column bottom component ($A_B$) of a lower portion of the high-boiling substance separation column A is supplied into the first continuous multistage distillation column and the second continuous multistage distillation column, whereby a catalyst necessary for a reaction is recycled.

The column bottom component ($A_B$) is rich in a high-boiling substance. Hence, in order to suppress elevation in the concentration of the high-boiling substance in the system, at least a portion of the column bottom component ($A_B$) is discarded without being recycled.

In the method described in Patent Document 1, both the first continuous multistage distillation column and the

second continuous multistage distillation column have insufficient reaction results. For producing the targeted diphenyl carbonate, it is necessary to circulate large amounts of the raw materials dialkyl carbonate and phenol. Hence, also for reducing circulation energy in the method described in Patent Document 1, the first continuous multistage distillation column and the second continuous multistage distillation column are required to enhance reaction results.

On the other hand, for enhancing reaction results in the method described in Patent Document 1, it may be possible to elevate a catalyst concentration in a lower liquid of the high-boiling substance separation column A. However, such elevation in catalyst concentration disadvantageously increases loss of the catalyst and therefore increases the replenishment of the catalyst.

Accordingly, an object of the present invention is to provide a method for producing diphenyl carbonate which can reduce a heat usage and reduce the amount of a catalyst replenished.

Means for Solving Problems

The present inventor has conducted studies to find a specific method that can attain the object described above, and consequently reached the present invention. Specifically, the present invention is as follows.

[1] A method for continuously producing diphenyl carbonate, comprising:

the step of continuously supplying dialkyl carbonate and phenol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl phenyl carbonate thus formed in a liquid form from a lower portion of the first continuous multistage distillation column to continuously produce alkyl phenyl carbonate;
the step of continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diphenyl carbonate thus formed in a liquid form from a lower portion of the second continuous multistage distillation column to continuously produce diphenyl carbonate;
a first purification step of continuously introducing the second column high-boiling reaction mixture containing diphenyl carbonate to a high-boiling substance separation column A, and continuously separating by distillation the mixture into a column top component ($A_T$) containing diphenyl carbonate and a column bottom component ($A_B$) containing the catalyst;
a second purification step of continuously introducing the column top component ($A_T$) to a diphenyl carbonate purifying column B having a side cut discharge port, and continuously separating by distillation the component into three components, a column top component ($B_T$), a side cut component ($B_S$), and a column bottom component ($B_B$), thereby obtaining diphenyl carbonate as the side cut component ($B_S$);
a first circulation step of continuously supplying the second column low-boiling reaction mixture into the first continuous multistage distillation column; and
a second circulation step of continuously supplying a portion of the column bottom component ($A_B$) into the first continuous multistage distillation column and/or into the second continuous multistage distillation column, wherein
the column bottom component ($A_B$) of the high-boiling substance separation column A contains diphenyl carbonate (DPC) and a high-boiling substance (HB), wherein a mass concentration ratio (DPC/HB) of the diphenyl carbonate to the high-boiling substance in the column bottom component ($A_B$) is 0.1 to 1.0, and a mass concentration ratio (DPC/catalyst) of the diphenyl carbonate to the catalyst is 1.0 to 10.0.

[2] The production method according to [1], wherein a concentration of the diphenyl carbonate in the column bottom component ($A_B$) is 9 to 38% by mass.
[3] The production method according to [1] or [2], wherein a material that is composed mainly of Fe and contains 2% by mass or more of Mo and 18% by mass or less of Cr is used in a liquid-contacting part in a lower portion of the second continuous multistage distillation column.
[4] The production method according to any of [1] to [3], wherein a concentration of alkyl aryl carbonate in the second column low-boiling reaction mixture is 1% by mass or less.
[5] The production method according to any of [1] to [4], wherein

(a) the first continuous multistage distillation column is a continuous multistage distillation column that comprises a cylindrical body with a length $L_1$ (cm) and an inside diameter $D_1$ (cm), comprises a structure having an internal having the number of stages $n_1$ in the inside, and comprises a gas discharge port with an inside diameter $d_{11}$ (cm) in a column top portion or an upper portion of the column close thereto, a liquid discharge port with an inside diameter $d_{12}$ (cm) in a column bottom portion or a lower portion of the column close thereto, one or more third introduction ports in an upper portion and/or an intermediate portion of the column below the gas discharge port, and one or more fourth introduction ports in an intermediate portion and/or a lower portion of the column above the liquid discharge port, wherein $L_1$, $D_1$, $L_1/D_1$, $n_1$, $D_1/d_{11}$, and $D_1/d_{12}$ respectively satisfy the following expressions (7) to (12):

$$1500 \leq L_1 \leq 8000 \quad \text{... Expression (7)}$$

$$100 \leq D_1 \leq 2000 \quad \text{... Expression (8)}$$

$$2 \leq L_1/D_1 \leq 40 \quad \text{... Expression (9)}$$

$$20 \leq n_1 \leq 120 \quad \text{... Expression (10)}$$

$$5 \leq D_1/d_{11} \leq 30 \quad \text{... Expression (11)}$$

$$3 \leq D_1/d_{12} \leq 20 \quad \text{... Expression (12);}$$

(b) the second continuous multistage distillation column is a continuous multistage distillation column that comprises a cylindrical body with a length $L_2$ (cm) and an inside diameter $D_2$ (cm), comprises a structure having an internal having the number of stages $n_2$ in the inside, and comprises a gas discharge port with an inside diameter $d_{21}$ (cm) in a column top portion or an upper portion of the column close thereto, a liquid discharge port with an inside diameter $d_{22}$ (cm) in a column bottom portion or a lower portion of the column close thereto, one or more fifth introduction ports in an upper portion and/or an intermediate portion of the column below the gas discharge port, and one or more sixth introduction ports in an intermediate portion and/or a lower portion of the column above the liquid discharge port, wherein $L_2$, $D_2$, $L_2/D_2$, $n_2$, $D_2/d_{22}$, and $D_2/d_{22}$ respectively satisfy the following expressions (13) to (18):

$$1500 \leq L_2 \leq 8000 \quad \text{... Expression (13)}$$

$$100 \leq D_2 \leq 2000 \quad \text{... Expression (14)}$$

$$2 \leq L_2/D_2 \leq 40 \quad \text{... Expression (15)}$$

$$10 \leq n_2 \leq 80 \quad \text{... Expression (16)}$$

$$2 \leq D_2/d_{21} \leq 15 \quad \text{... Expression (17)}$$

$$5 \leq D_2/d_{22} \leq 30 \quad \text{... Expression (18);}$$

(c) the high-boiling substance separation column A is a continuous multistage distillation column that comprises a cylindrical body with a length $L_A$ (cm) and an inside diameter $D_A$ (cm) and comprises an internal having the

number of stages $n_A$ in the inside, wherein $L_A$, $D_A$, and $n_A$ respectively satisfy the following expressions (19) to (21) :

$$800 \leq L_A \leq 3000 \quad \text{... Expression (19)}$$

$$100 \leq D_A \leq 1000 \quad \text{... Expression (20)}$$

$$20 \leq n_A \leq 100 \quad \text{... Expression (21);}$$

and

(d) the diphenyl carbonate purifying column B is a continuous multistage distillation column that comprises a cylindrical body with a length $L_B$ (cm) and an inside diameter $D_B$ (cm), comprises a structure having an internal in the inside, and comprises an introduction port $B_1$ in an intermediate portion of the column and a side cut discharge port $B_2$ between the introduction port $B_1$ and the column bottom, wherein the number of stages of the internal from the introduction port $B_1$ through an upper portion is defined as $n_{B1}$, the number of stages of the internal between the introduction port $B_1$ and the side cut discharge port $B_2$ is defined as $n_{B2}$, the number of stages of the internal from the side cut discharge port $B_2$ through a lower portion is defined as $n_{B3}$, and the total number of stages ($n_B$, + $n_{B2}$ + $n_{B3}$) is defined as $n_B$, wherein $L_B$, $D_B$, $n_{B1}$, $n_{B2}$, $n_{B3}$, and $n_B$ respectively satisfy the following expressions (22) to (27):

$$1000 \leq L_B \leq 5000 \quad \text{... Expression (22)}$$

$$100 \leq D_B \leq 1000 \quad \text{... Expression (23)}$$

$$5 \leq n_{B1} \leq 20 \quad \text{... Expression (24)}$$

$$12 \leq n_{B2} \leq 40 \quad \text{... Expression (25)}$$

$$3 \leq n_{B3} \leq 15 \quad \text{... Expression (26)}$$

$$20 \leq n_B \leq 70 \quad \text{... Expression (27).}$$

Advantages of Invention

The present invention can provide a method for producing diphenyl carbonate which can reduce a heat usage and reduce the amount of a catalyst replenished.

Brief Description of Drawings

[Figure 1] Figure 1 is a schematic view showing one example of a continuous multistage distillation column $T_0$. An internal formed from a sieve tray is installed in the inside of a body.

[Figure 2] Figure 2 is a schematic view showing one example of a first continuous multistage distillation column. An internal is installed in the inside of a body.

[Figure 3] Figure 3 is a schematic view showing one example of a second continuous multistage distillation column. A structured packing is installed in an upper portion, and an internal formed from a sieve tray is installed in a lower portion, in the inside of a body.

[Figure 4] Figure 4 is a schematic view showing one example of an apparatus comprising a first continuous multistage

distillation column connected with a second continuous multistage distillation column.

[Figure 5] Figure 5 is a schematic view showing one example of an apparatus comprising a high-boiling substance separation column A connected with a diphenyl carbonate purifying column B.

Mode for Carrying Out Invention

Hereinafter, the mode for carrying out the present invention (hereinafter, referred to as the "present embodiment") will be described in detail with reference to the drawings, if necessary. However, the present invention is not limited thereby, and various changes or modifications can be made therein without departing from the spirit of the present invention. In the drawings, positional relationships indicated by terms such as "up", "down", "right", and "left" are based on the positional relationships shown in the drawings, unless otherwise specified. Dimensional ratios in the drawings are not limited to the shown ratios.

The method for producing diphenyl carbonate according to the present embodiment is a method for continuously producing diphenyl carbonate, comprising:

the step of continuously supplying dialkyl carbonate and phenol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl phenyl carbonate thus formed in a liquid form from a lower portion of the first continuous multistage distillation column to continuously produce alkyl phenyl carbonate;

the step of continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diphenyl carbonate thus formed in a liquid form from a lower portion of the second continuous multistage distillation column to continuously produce diphenyl carbonate;

a first purification step of continuously introducing the second column high-boiling reaction mixture containing diphenyl carbonate to a high-boiling substance separation column A, and continuously separating by distillation the mixture into a column top component ($A_T$) containing diphenyl carbonate and a column bottom component ($A_B$) containing the catalyst;

a second purification step of continuously introducing the column top component ($A_T$) to a diphenyl carbonate purifying column B having a side cut discharge port, and continuously separating by distillation the component into three components, a column top component ($B_T$), a side cut component ($B_S$), and a column bottom component ($B_B$), thereby obtaining diphenyl carbonate as the side cut component ($B_S$);

a first circulation step of continuously supplying the second column low-boiling reaction mixture into the first continuous multistage distillation column; and

a second circulation step of continuously supplying a portion of the column bottom component ($A_B$) into the first continuous multistage distillation column and/or into the second continuous multistage distillation column, wherein the column bottom component ($A_B$) of the high-boiling substance separation column A contains diphenyl carbonate (hereinafter, also referred to as "DPC") and a high-boiling substance (hereinafter, also referred to as "HB"), wherein a mass concentration ratio (DPC/HB) of the diphenyl carbonate to the high-boiling substance in the column bottom component ($A_B$) is 0.1 to 1.0, and a mass concentration ratio (DPC/catalyst) of the diphenyl carbonate to the catalyst is 1.0 to 10.0.

Owing to the features as described above, the method for producing diphenyl carbonate according to the present embodiment exerts effects of being able to reduce a heat usage and reduce the amount of a catalyst replenished. Although a mechanism under which such effects are exerted is not certain, the present inventor deduces the following mechanism.

The column bottom component ($A_B$) of the high-boiling substance separation column A is recycled to the first continuous multistage distillation column and the second continuous multistage distillation column and usually contains not only the catalyst but diphenyl carbonate, a high-boiling substance, and the like. The method for producing diphenyl carbonate according to the present embodiment controls the mass concentration ratio (DPC/HB) of the diphenyl carbonate to the high-boiling substance in the column bottom component ($A_B$) to a predetermined range as described above by elevating the concentration of the high-boiling substance in the column bottom component ($A_B$), and thereby lowering the concentration of the diphenyl carbonate contained in a recycled liquid.

This provides an advantage to the formulation of the diphenyl carbonate in equilibrium reaction, particularly, in

the second continuous multistage distillation column. Thus, the method for producing diphenyl carbonate according to the present embodiment can presumably improve reaction results. On the other hand, if the mass concentration ratio (DPC/HB) of the diphenyl carbonate to the high-boiling substance in the column bottom component (AB) is decreased too much, evaporation in a reboiler is impossible because of too high a column bottom temperature of the high-boiling substance separation column. From such a viewpoint, the mass concentration ratio (DPC/HB) of the diphenyl carbonate to the high-boiling substance in the column bottom component (AB) is preferably 0.2 to 0.8, more preferably 0.3 to 0.6.

As described above, the mass concentration ratio (DPC/catalyst) of the diphenyl carbonate to the catalyst in the column bottom component ($A_B$) is controlled to a predetermined range, whereby the reaction results can presumably be improved while the amount of a catalyst replenished is suppressed. From such a viewpoint, the mass concentration ratio (DPC/catalyst) of the diphenyl carbonate to the catalyst in the column bottom component ($A_B$) is preferably 7.0 to 9.5, more preferably 8.0 to 9.0.

The concentration of the diphenyl carbonate in the column bottom component ($A_B$) is preferably 9 to 38% by mass, more preferably 19 to 36% by mass, further preferably 28 to 34% by mass. When the concentration of the diphenyl carbonate in the column bottom component ($A_B$) falls within the range described above, there is a tendency to be able to more reduce a heat usage and to more reduce the amount of a catalyst replenished.

The method for producing diphenyl carbonate according to the present embodiment is also capable of decreasing, for example, the amount of dialkyl carbonate and phenol circulated, by improving reaction results. This can also reduce an energy usage in the first circulation step.

In the first circulation step, circulation is formed, for example, by heating dialkyl carbonate and phenol so as to climb to the column top using vapor in the first continuous multistage distillation column and the second continuous multistage distillation column. Therefore, the amount of these components circulated can be decreased, and the energy usages of the first continuous multistage distillation column and the second continuous multistage distillation column can thereby be reduced.

For adjusting the mass concentration ratio (DPC/HB) of the diphenyl carbonate to the high-boiling substance in the column bottom component ($A_B$) of the high-boiling substance separation column A to 0.1 to 1.0, it is necessary to heat DPC of the column bottom so as to climb to the column top, as compared with a conventional method. Therefore, the mass concentration ratio (DPC/HB) can be adjusted to within the range mentioned above, for example, by rendering the pressure of the high-boiling substance separation column lower than that of the conventional method.

For adjusting the mass concentration ratio (DPC/catalyst) of the diphenyl carbonate to the catalyst in the column bottom component ($A_B$) of the high-boiling substance separation column to 1.0 to 10.0, it is usually possible to decrease the abundance of the catalyst. When the abundance of the catalyst is decreased, the mass concentration ratio (DPC/HB) mentioned above tends to be increased whereas reactions in the first continuous multistage distillation column and the second continuous multistage distillation column are reduced. Therefore, the mass concentration ratio (DPC/catalyst) can be adjusted to within the range mentioned above, for example, by lowering a DPC concentration instead of decreasing the abundance of the catalyst.

In the present embodiment, the concentrations of the diphenyl carbonate (DPC), the high-boiling substance (HB), and the catalyst in the column bottom component ($A_B$) can be measured by methods described in Examples mentioned later.

In the present embodiment, the high-boiling substance refers to a substance having a higher boiling point than that of diphenyl carbonate and excludes a catalyst.

In the present embodiment, the method for obtaining the raw material dialkyl carbonate is not particularly limited, and, for example, a step (I) of continuously producing dialkyl carbonate and diols at an industrial scale from cyclic carbonate and aliphatic monovalent alcohols may be performed. The reaction of the step (I) is reversible transesterification reaction represented by the following formula:

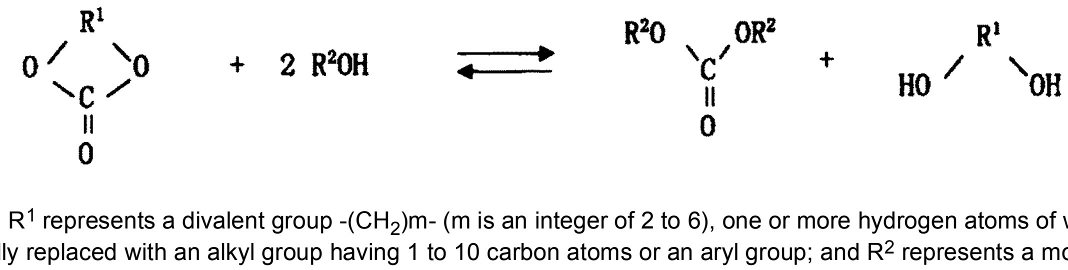

wherein $R^1$ represents a divalent group $-(CH_2)m-$ (m is an integer of 2 to 6), one or more hydrogen atoms of which are optionally replaced with an alkyl group having 1 to 10 carbon atoms or an aryl group; and $R^2$ represents a monovalent aliphatic group having 1 to 12 carbon atoms, one or more hydrogen atoms of which are optionally replaced with an alkyl group having 1 to 10 carbon atoms or an aryl group.

Examples of such cyclic carbonate that is preferably used include alkylene carbonates such as ethylene carbonate and propylene carbonate, 1,3-dioxacyclohex-2-one, and 1,3-dioxacyclohept-2-one. Ethylene carbonate and propylene carbonate are further preferably used from the viewpoint of easy availability, etc., and ethylene carbonate is particularly preferably used.

The aliphatic monovalent alcohols used have a lower boiling point than that of diols to be formed. Thus, examples of the aliphatic monovalent alcohols, which may vary depending on the type of the cyclic carbonate used, include methanol, ethanol, propanol (each isomer), allyl alcohol, butanol (each isomer), 3-buten-1-ol, amyl alcohol (each isomer), hexyl alcohol (each isomer), heptyl alcohol (each isomer), octyl alcohol (each isomer), nonyl alcohol (each isomer), decyl alcohol (each isomer), undecyl alcohol (each isomer), dodecyl alcohol (each isomer), cyclopentanol, cyclohexanol, cycloheptanol, cyclooctanol, methylcyclopentanol (each isomer), ethylcyclopentanol (each isomer), methylcyclohexanol (each isomer), ethylcyclohexanol (each isomer), dimethylcyclohexanol (each isomer), diethylcyclohexanol (each isomer), phenylcyclohexanol (each isomer), benzyl alcohol, phenethyl alcohol (each isomer), and phenylpropanol (each isomer). These aliphatic monovalent alcohols may be substituted by substituents such as halogen, a lower alkoxy group, a cyano group, an alkoxycarbonyl group, an aryloxycarbonyl group, an acyloxy group, or a nitro group.

Among such aliphatic monovalent alcohols, alcohols having 1 to 6 carbon atoms are preferably used, and alcohols having 1 to 4 carbon atoms, i.e., methanol, ethanol, propanol (each isomer), and butanol (each isomer), are further preferred. In the case of using ethylene carbonate or propylene carbonate as cyclic carbonate, methanol and ethanol are preferred, and methanol is particularly preferred.

The method for allowing the reactive distillation column to contain a catalyst in performing reactive distillation in the step (I) can be any method. In the case of, for example, a homogeneous catalyst soluble in a reaction liquid under reaction conditions, a liquid phase in the reactive distillation column can be allowed to contain the catalyst by continuously supplying the catalyst into the reactive distillation column. Alternatively, in the case of an inhomogeneous catalyst insoluble in a reaction liquid under reaction conditions, the reaction system can be allowed to contain the catalyst by placing a solid catalyst in the reactive distillation column. These methods may be used in combination.

The homogeneous catalyst, when continuously supplied into the reactive distillation column, may be supplied simultaneously with the cyclic carbonate and/or the aliphatic monovalent alcohol or may be supplied to a position different from that for raw materials. A region where the reaction actually progresses in the distillation column is located below a catalyst supply position. It is therefore preferred to supply the catalyst to a region between the column top and a portion slightly lower than a raw material supply position. Plates containing the catalyst are preferably five or more plates, more preferably seven or more plates, further preferably ten or more plates.

In the case of using an inhomogeneous solid catalyst, the number of plates containing the catalyst is preferably 5 or more, more preferably 7 or more, further preferably 10 or more. A solid catalyst also having effects as a distillation column packing may be used.

Examples of the catalyst for use in the step (I) include, but are not particularly limited to:

alkali metals and alkaline earth metals such as lithium, sodium, potassium, rubidium, cesium, magnesium, calcium, strontium, and barium;
basic compounds such as hydride, hydroxide, alkoxides, aryloxides, and amides of alkali metals and alkaline earth metals;
basic compounds such as carbonates, bicarbonates, and organic acid salts of alkali metals and alkaline earth metals;
tertiary amines such as triethylamine, tributylamine, trihexylamine, and benzyldiethylamine;
nitrogen-containing heteroaromatic compounds such as N-alkylpyrrole, N-alkylindole, oxazole, N-alkylimidazole, N-alkylpyrazole, oxadiazole, pyridine, alkylpyridine, quinoline, alkylquinoline, isoquinoline, alkylisoquinoline, acridine, alkylacridine, phenanthroline, alkylphenanthroline, pyrimidine, alkylpyrimidine, pyrazine, alkylpyrazine, triazine, and alkyltriazine;
cyclic amidines such as diazabicycloundecene (DBU) and diazabicyclononene (DBN);
thallium compounds such as thallium oxide, thallium halide, thallium hydroxide, thallium carbonate, thallium nitrate, thallium sulfate, and organic acid salts of thallium;
tin compounds such as tributylmethoxytin, tributylethoxytin, dibutyldimethoxytin, diethyldiethoxytin, dibutyldiethoxytin, dibutylphenoxytin, diphenylmethoxytin, dibutyltin acetate, tributyltin chloride, and tin 2-ethylhexanoate;
zinc compounds such as dimethoxyzinc, diethoxyzinc, ethylenedioxyzinc, and dibutoxyzinc;
aluminum compounds such as aluminum trimethoxide, aluminum triisopropoxide, and aluminum tributoxide;
titanium compounds such as tetramethoxytitanium, tetraethoxytitanium, tetrabutoxytitanium, dichlorodimethoxytitanium, tetraisopropoxytitanium, titanium acetate, and titanium acetylacetonate;
phosphorus compounds such as trimethylphosphine, triethylphosphine, tributylphosphine, triphenylphosphine, tributylmethyl phosphonium halide, trioctylbutyl phosphonium halide, and triphenylmethyl phosphonium halide;
zirconium compounds such as zirconium halide, zirconium acetylacetonate, zirconium alkoxide, and zirconium acetate;
lead and compounds containing lead, for example, lead oxides such as PbO, $PbO_2$, and $Pb_3O_4$;
lead sulfides such as PbS, $Pb_2S_3$, and $PbS_2$;

lead hydroxides such as $Pb(OH)_2$, $Pb_3O_2(OH)_2$, $Pb_2[PbO_2(OH)_2]$, and $Pb_2O(OH)_2$;
plumbites such as $Na_2PbO_2$, $K_2PbO_2$, $NaHPbO_2$, and $KHPbO_2$ ;
plumbates such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$;
carbonate of lead and basic salts thereof, such as $PbCO_3$ and $2PbCO_3 \cdot Pb(OH)_2$;
alkoxyleads and aryloxyleads such as $Pb(OCH_3)_2$, $(CH_3O)Pb(OPh)$, and $Pb(OPh)_2$;
lead salts of organic acids and carbonate and basic salts thereof, such as $Pb(OCOCH_3)_2$, $Pb(OCOCH_3)_4$, and $Pb(OCOCH_3)_2$-PbO-$3H_2O$;
organic lead compounds such as $Bu_4Pb$, $Ph_4Pb$, $Bu_3PbCl$, $Ph_3PbBr$, $Ph_3Pb$ (or $Ph_6Pb_2$), $Bu_3PbOH$, and $Ph_2PbO$ (Bu represents a butyl group, and Ph represents a phenyl group);
alloys of lead such as Pb-Na, Pb-Ca, Pb-Ba, Pb-Sn, and Pb-Sb; and
lead minerals such as galena and sphalerite, and hydrides of these lead compounds.

These compounds can be used as homogeneous catalysts when soluble in reaction raw materials, a reaction mixture, reaction by-products, or the like, and can be used as solid catalysts when insoluble therein. These compounds are dissolved beforehand in reaction raw materials, a reaction mixture, reaction by-products, or the like, or use of a mixture dissolved through a reaction as a homogeneous catalyst is also a preferred method.

Further examples of the catalyst used include: ion exchangers such as anion-exchange resins having a tertiary amino group, ion-exchange resins having an amide group, ion-exchange resins having at least one exchange group selected from a sulfonic acid group, a carboxylic acid group, and a phosphoric acid group, and solid strongly basic anion exchangers having a quaternary ammonium group as an exchange group; and solid inorganic compounds such as silica, silica-alumina, silicamagnesia, aluminosilicate, gallium silicate, various zeolites, various metal-exchanged zeolites, and ammoniumexchanged zeolites.

A solid strongly basic anion exchanger having a quaternary ammonium group as an exchange group is particularly preferably used as a solid catalyst. Examples of such a solid strongly basic anion exchanger include, but are not particularly limited to, strongly basic anion-exchange resins having a quaternary ammonium group as an exchange group, cellulose strongly basic anion exchangers having a quaternary ammonium group as an exchange group, and strongly basic anion exchangers of type supported on an inorganic support having a quaternary ammonium group as an exchange group. The strongly basic anion-exchange resin having a quaternary ammonium group as an exchange group is not particularly limited, and, for example, a styrenic strongly basic anion-exchange resin is preferably used. The styrenic strongly basic anion-exchange resin is a strongly basic anion-exchange resin having a copolymer of styrene and divinylbenzene as a matrix and having quaternary ammonium (type I or type II) as an exchange group, and is schematically represented by, for example, the following formula:

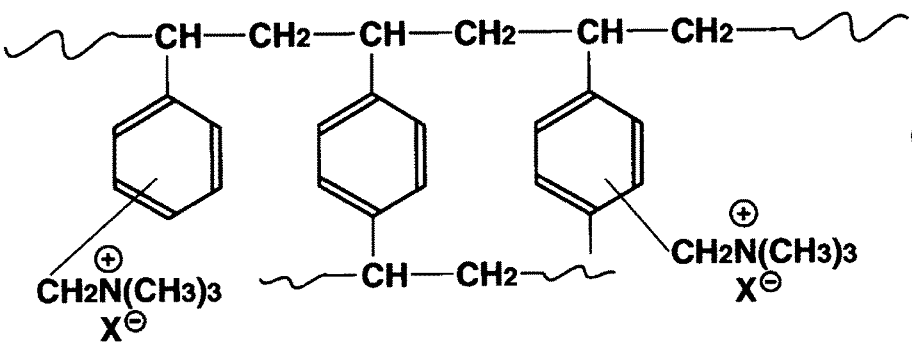

(type I)

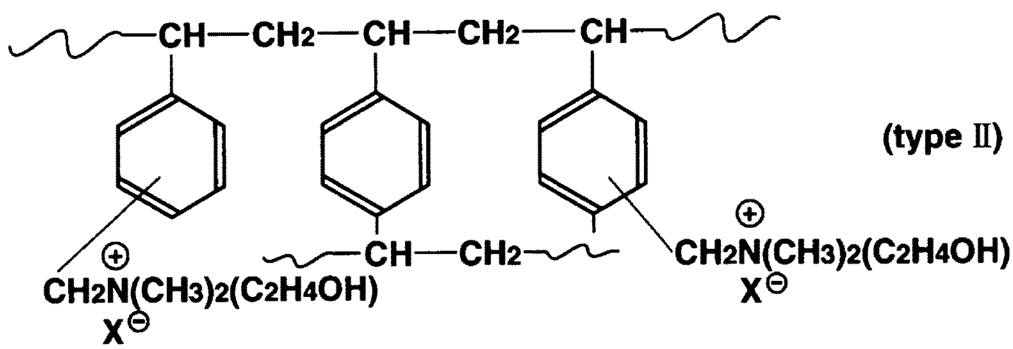

(type II)

In the formula, X represents an anion. At least one anion selected from among $F^-$, $Cl^-$, $Br^-$, $I^-$, $HCO_3^-$, $CO_3^{2-}$,

$CH_3CO_2^-$, $HCO_2^-$, $IO_3^-$, $BrO_3^-$, and $ClO_3^-$ is usually used as X, and at least one anion selected from among $Cl^-$, $Br^-$, $HCO_3^-$, and $CO_3^{2-}$ is preferably used. Any of gel type and macroreticular type (MR type) can be used as the structure of the resin matrix, and MR type is particularly preferred from the viewpoint of high organic solvent resistance.

Examples of the cellulose strongly basic anion exchanger having a quaternary ammonium group as an exchange group include, but are not particularly limited to, cellulose having an exchange group $-OCH_2CH_2NR_3X$ obtained by trialkylaminoethylating some or all of -OH groups of cellulose. In the formula, R represents an alkyl group. Methyl, ethyl, propyl, butyl, or the like is usually used, and methyl or ethyl is preferably used. X represents an anion, as mentioned above.

The strongly basic anion exchanger of type supported on an inorganic support having a quaternary ammonium group as an exchange group means an anion exchanger in which a quaternary ammonium group - $O(CH_2)nNR_3X$ is introduced by modifying some or all of surface hydroxy groups -OH of an inorganic support. In the formula, R and X are as mentioned above, n is usually an integer of 1 to 6, preferably n = 2. The inorganic support is not particularly limited, and, for example, silica, alumina, silica-alumina, titania, or zeolite can be used. Silica, alumina, or silica-alumina is preferably used, and silica is particularly preferably used. An arbitrary method can be used as a method for modifying the surface hydroxy groups of an inorganic support.

A commercially available product can also be used as the solid strongly basic anion exchanger having a quaternary ammonium group as an exchange group. In this case, ion exchange may be performed with the desired anion species in pretreatment, and the resultant can then be used as a transesterification catalyst.

An organic polymer of macroreticular or gel type bonded to a heterocyclic group containing at least one nitrogen atom, or a solid catalyst formed from an inorganic support bonded to a heterocyclic group containing at least one nitrogen atom is also preferably used as a transesterification catalyst. Further, a solid catalyst in which some or all of such nitrogen-containing heterocyclic groups are converted into a quaternary salt is similarly used. A solid catalyst such as an ion exchanger can also function as a packing.

The amount of the catalyst used in the step (I) differs depending on the type of the catalyst used. In the case of continuously supplying a homogeneous catalyst soluble in a reaction liquid under reaction conditions, the catalyst is used at usually 0.0001 to 50% by mass, preferably 0.005 to 20% by mass, further preferably 0.01 to 10% by mass, in terms of a ratio to the total mass of the cyclic carbonate and the aliphatic monovalent alcohol serving as supplied raw materials. In the case of using a solid catalyst installed in the distillation column, the catalyst is preferably used in an amount of 0.01 to 75% by volume, preferably 0.05 to 60% by volume, further preferably 0.1 to 60% by volume, based on the vacant column volume of the distillation column.

In the step (I), the method for continuously supplying the raw materials cyclic carbonate and the aliphatic monovalent alcohol into a continuous multistage distillation column $T_0$ which is a reactive distillation column is not particularly limited and can be any method as long as the supply method can bring the raw materials into contact with a catalyst, for example, in a region of at least five or more plates, preferably seven or more plates, more preferably ten or more plates, of the distillation column. Specifically, the cyclic carbonate and the aliphatic monovalent alcohol can be continuously supplied from a necessary number of introduction ports for plates that satisfy the conditions described above in the continuous multistage distillation column. The cyclic carbonate and the aliphatic monovalent alcohol may be introduced to the same plates of the distillation column or may be separately introduced to different plates.

The raw materials cyclic carbonate and aliphatic monovalent alcohol are continuously supplied in a liquid form, in a gas form, or as a mixture of a liquid and a gas into the continuous multistage distillation column $T_0$. In addition to such supply of the raw materials into the distillation column, the raw materials may be additionally supplied intermittently or continuously in a gas form from a lower portion of the distillation column, and this is also a preferred method. A method of continuously supplying the cyclic carbonate in a liquid form or in a gas-liquid mixed state to plates above plates containing a catalyst in the distillation column, and continuously supplying the aliphatic monovalent alcohol in a gas form and/or in a liquid form to a lower portion of the distillation column is also a preferred method. In this case, the cyclic carbonate may contain the aliphatic monovalent alcohol, as a matter of course.

In the step (I), the supplied raw materials may contain the products dialkyl carbonate and/or diols. The contents thereof are usually 0 to 40% by mass, preferably 0 to 30% by mass, further preferably 0 to 20% by mass, of the dialkyl carbonate in terms of % by mass in an aliphatic monovalent alcohol/dialkyl carbonate mixture, and usually 0 to 10% by mass, preferably 0 to 7% by mass, further preferably 0 to 5% by mass, of the diols in terms of % by mass in a cyclic carbonate/diol mixture.

In the case of industrially carrying out the reaction of the step (I), it is preferred that cyclic carbonate and/or aliphatic monovalent alcohols that are newly introduced to the reaction system as well as a substance composed mainly of cyclic carbonate and/or aliphatic monovalent alcohols recovered in this step and/or other steps should be able to be used as raw materials. Other steps include, for example, a step (II) of producing diphenyl carbonate from dialkyl carbonate and phenol, and in this step (II), aliphatic monovalent alcohols are recovered as by-products. The aliphatic monovalent alcohols recovered as by-products often contain dialkyl carbonate, phenol, alkyl aryl ether, and the like and may further contain a small amount of alkyl aryl carbonate, diphenyl carbonate, and the like. The by-products aliphatic monovalent alcohols may be used directly as raw materials in the step (I) or may be used as raw materials in the step (I) after the

content of substances having a higher boiling point than that of the aliphatic monovalent alcohols is decreased by distillation or the like.

The preferred cyclic carbonate for use in the step (I) is produced, for example, through the reaction of alkylene oxide such as ethylene oxide, propylene oxide, or styrene oxide with carbon dioxide. Therefore, cyclic carbonate containing a small amount of these compounds, etc. may be used as raw materials in the step (I).

In the step (I), the quantitative ratio between the cyclic carbonate and the aliphatic monovalent alcohols to be supplied to the reactive distillation column differs depending on the type and amount of a transesterification catalyst and reaction conditions. Usually, the aliphatic monovalent alcohols can be supplied in the range of 0.01 to 1000 times the mol of the cyclic carbonate to be supplied. For enhancing the reaction ratio of the cyclic carbonate, it is preferred to supply the aliphatic monovalent alcohols in an excessive amount of twice or more the mol of the cyclic carbonate, though use of the aliphatic monovalent alcohols in a large excess requires increasing the size of an apparatus. In this respect, the molar ratio of the aliphatic monovalent alcohols to the cyclic carbonate is preferably 2 to 20, more preferably 3 to 15, further preferably 5 to 12. If unreacted cyclic carbonate remains in a large amount, this cyclic carbonate reacts with the products diols to form multimers such as dimers or trimers as by-products. Therefore, in the case of carrying out an industrial process, it is preferred to minimize the amount of unreacted cyclic carbonate remaining.

In the step (I), for example, in the case of continuously producing approximately 0.4 tons or more of dialkyl carbonate per hour, the lowest amount of the cyclic carbonate continuously supplied is usually 0.44P ton/hr, preferably 0.42P ton/hr, more preferably 0.4P ton/hr, based on the amount (P ton/hr) of the dialkyl carbonate to be produced. Further preferably, the lowest amount can be smaller than 0.39P ton/hr.

The continuous multistage distillation column $T_0$ for use in the step (I) is not particularly limited and is, for example, a continuous multistage distillation column as shown in Figure 1 that comprises a cylindrical body with a length $L_0$ (cm) and an inside diameter $D_0$ (cm), comprises a structure having an internal having the number of stages $n_0$ in the inside, and comprises a gas discharge port with an inside diameter $d_{01}$, (cm) in a column top portion or an upper portion of the column close thereto, a liquid discharge port with an inside diameter $d_{02}$ (cm) in a column bottom portion or a lower portion of the column close thereto, one or more first introduction ports in an upper portion and/or an intermediate portion of the column below the gas discharge port, and one or more second introduction ports in an intermediate portion and/or a lower portion of the column above the liquid discharge port, wherein $L_0$, $D_0$, $L_0/D_0$, $n_0$, $D_0/d_{01}$, and $D_0/d_{02}$ respectively satisfy the following expressions (1) to (6):

$$2100 \leq L_0 \leq 8000 \quad \ldots \text{ Expression (1)}$$

$$180 \leq D_0 \leq 2000 \quad \ldots \text{ Expression (2)}$$

$$4 \leq L_0/D_0 \leq 40 \quad \ldots \text{ Expression (3)}$$

$$10 \leq n_0 \leq 120 \quad \ldots \text{ Expression (4)}$$

$$3 \leq D_0/d_{01} \leq 20 \quad \ldots \text{ Expression (5)}$$

$$5 \leq D_0/d_{02} \leq 30 \quad \ldots \text{ Expression (6).}$$

The term "column top portion or upper portion of the column close thereto" used in the present embodiment means a portion from the column top portion to approximately $0.25L_0$ downward, and the term "column bottom portion or lower portion of the column close thereto" means a portion from the column bottom portion to approximately $0.25L_0$ upward (these correspond to $0.25L_1$ and $0.25L_2$, respectively, in the first and second continuous multistage distillation columns, and $0.25L_A$ and $0.25L_B$, respectively, in the high-boiling substance separation column A and the diphenyl carbonate purifying column B).

By using the continuous multistage distillation column $T_0$ that simultaneously satisfies the expressions (1), (2), (3), (4), (5), and (6), dialkyl carbonate and/or diols can be stably produced from cyclic carbonate and aliphatic monovalent alcohols with high reaction ratio, high selectivity, and high productivity at an industrial scale of preferably 0.4 tons or more of the dialkyl carbonate per hour and preferably 0.26 tons or more of the diols per hour for a long period of, for example, 1000 hours or longer, preferably 3000 hours or longer, further preferably 5000 hours or longer. The reason is

not clear why the step (I) allows for the production of dialkyl carbonate and diols at an industrial scale having such excellent effects. The effects are presumably combined effects brought about by a combination of the conditions of the expressions (1) to (6). Preferred ranges of the respective factors will be given below.

When $L_0$ (cm) is 2100 or more, reaction ratio is favorable and the desired production amount can be achieved. For reducing the cost of equipment while securing the reaction ratio that can achieve the desired production amount, $L_0$ is preferably 8000 or less. $L_0$ (cm) is more preferably in the range of $2300 \leq L_0 \leq 6000$, further preferably $2500 \leq L_0 \leq 5000$.

When $D_0$ (cm) is 180 or more, the desired production amount can be achieved. For reducing the cost of equipment while achieving the desired production amount, $D_0$ is preferably 2000 or less. $D_0$ (cm) is more preferably in the range of $200 \leq D_0 \leq 1000$, further preferably $210 \leq D_0 \leq 800$.

When $L_0/D_0$ is 4 or more or is 40 or less, stable operation is easy to perform. Particularly, when $L_0/D_0$ is 40 or less, long-term stable operation is easy to perform because difference in pressure between the upper and lower portions of the column can be prevented from becoming too large. Besides, side reaction can be suppressed, and selectivity tends to be improved, because a temperature in the lower portion of the column can be decreased. $L_0/D_0$ is more preferably in the range of $5 \leq L_0/D_0 \leq 30$, further preferably $7 \leq L_0/D_0 \leq 20$.

When $n_0$ is 10 or more, the desired production amount can be achieved because reaction ratio is improved. For reducing the cost of equipment while securing the reaction ratio that can achieve the desired production amount, $n_0$ is preferably 120 or less. When $n_0$ is 120 or less, long-term stable operation is easy to perform because difference in pressure between the upper and lower portions of the column can be prevented from becoming too large. Besides, side reaction can be suppressed, and selectivity tends to be improved, because a temperature in the lower portion of the column can be decreased. $n_0$ is more preferably in the range of $30 \leq n_0 \leq 100$, further preferably $40 \leq n_0 \leq 90$.

When $D_0/d_{01}$ is 3 or more, the cost of equipment can be reduced. Besides, a gas component can be prevented from coming out of the system and stable operation is easy to perform. When the ratio is 20 or less, the amount of a gas component discharged is relatively large and stable operation is easy to perform. Besides reaction ratio tends to be improved. $D_0/d_{01}$ is more preferably in the range of $4 \leq D_0/d_{01} \leq 15$, further preferably $5 \leq D_0/d_{01} \leq 13$.

When $D_0/d_{02}$ is 5 or more, the cost of equipment can be reduced. Besides, the amount of a liquid discharged is relatively small and stable operation is easy to perform. When the ratio is 30 or less, a flow rate in a liquid discharge port or a piping can be prevented from being rapidly accelerated, erosion is unlikely to occur, and the corrosion of an apparatus can be suppressed. $D_0/d_{02}$ is more preferably in the range of $7 \leq D_0/d_{02} \leq 25$, further preferably $9 \leq D_0/d_{02} \leq 20$.

The factors $d_{01}$ and $d_{02}$ of the continuous multistage distillation column $T_0$ for use in the step (I) further preferably satisfy the expression (28):

$$1 \leq d_{01}/d_{02} \leq 5 \quad \text{... Expression (28).}$$

The long-term stable operation described in the step (I) means that operation can be continued for 1000 hours or longer, preferably 3000 hours or longer, further preferably 5000 hours or longer, in a steady state based on operation conditions without flooding or piping clogging or erosion, and predetermined amounts of dialkyl carbonate and diols are produced while high reaction ratio, high selectivity, and high productivity are maintained.

The selectivity of dialkyl carbonate and diols described in the step (I) is based on the reacted cyclic carbonate. In the present embodiment, high selectivity of usually 95% or more, preferably high selectivity of 97% or more, more preferably 99% or more, can be achieved. The reaction ratio described in the step (I) usually refers to the reaction ratio of the cyclic carbonate. In the present embodiment, the reaction ratio of the cyclic carbonate can be 95% or more, preferably 97% or more, more preferably 99% or more, further preferably 99.5 or more, still further preferably 99.9% or more. Such high reaction ratio that can be achieved with high selectivity maintained is also one of the excellent features of the step (I).

The continuous multistage distillation column $T_0$ for use in the step (I) is preferably a distillation column having a tray and/or a packing as the internal. The internal described in the present embodiment means a portion where the contact between a gas and a liquid is actually performed in the distillation column. Such a tray is not particularly limited and is preferably, for example, a bubble-cap tray, a sieve tray, a valve tray, a countercurrent tray, a Superfrac tray, or a Max-Frac tray, and the packing is preferably a random packing such as a Raschig ring, a Lessing ring, a pall ring, a Berl saddle, an Intalox saddle, Dixon Packing, Mc MAHON Packing, or Heli Pack, or a structured packing such as Mellapak, Gempak, Techno Pack, Flexipac, Sulzer Packings, Goodloe Packing, or Glitsch Grid. A multistage distillation column having both of a tray portion and a portion filled with a packing can also be used. The term "number of stages of the internal" described in the present embodiment means the number of trays in the case of a tray and means the theoretical number of stages in the case of a packing. Thus, in the case of a multistage distillation column having both of a tray portion and a portion filled with a packing, the number of stages is the total sum of the number of trays and the theoretical number of stages.

In the step (I) of reacting cyclic carbonate with aliphatic monovalent alcohols, use of any of a continuous

multistage distillation column of plate column type and a continuous multistage distillation column of packed column type having a tray and/or a packing having a predetermined number of stages in the internal tends to be able to achieve high reaction ratio, high selectivity, and high productivity. The distillation column of plate column type having a tray in the internal is more preferred. The tray is a sieve tray having a sieve portion and a downcomer portion because of an excellent relationship between functions and the cost of equipment. The sieve tray preferably has 100 to 1000 holes per $m^2$ of the area of the sieve portion. The number of holes is more preferably 120 to 900, further preferably 150 to 800, per $m^2$ of the area. The cross section per hole of the sieve tray is preferably 0.5 to 5 $cm^2$. The cross section per hole is more preferably 0.7 to 4 $cm^2$, further preferably 0.9 to 3 $cm^2$. The case is particularly preferred where the sieve tray has 100 to 1000 holes per $m^2$ of the area of the sieve portion, and the cross section per hole is 0.5 to 5 $cm^2$.

The aperture ratio of the sieve tray is preferably 1.5 to 15%. The aperture ratio is more preferably 1.7 to 130, further preferably 1.9 to 11%. In this context, the aperture ratio of the sieve tray refers to the ratio of the cross section of all holes (total hole cross section) present in a sieve to the area (including the total hole cross section) of the sieve portion. The area and/or total hole cross section of the sieve portion may differ among sieve trays. In this case as well, the aperture ratio of each sieve tray preferably falls within the range described above. The number of holes in the sieve portion may be the same among all sieves or may be different.

The step (I), when carried out, involves, for example, continuously supplying the raw materials cyclic carbonate and aliphatic monovalent alcohols into a continuous multistage distillation column containing a catalyst, simultaneously performing a reaction and distillation in the column, continuously discharging a low-boiling reaction mixture containing dialkyl carbonate thus formed in a gas form from an upper portion of the column, and continuously discharging a high-boiling reaction mixture containing diols in a liquid form from a lower portion of the column to continuously produce dialkyl carbonate and diols.

In the step (I), the raw materials cyclic carbonate and aliphatic monovalent alcohols, when continuously supplied into the continuous multistage distillation column $T_0$, may be supplied in a liquid form and/or in a gas form either as a raw material mixture or separately from an introduction port installed at one or several locations below an upper gas discharge port of the distillation column and in an upper portion or an intermediate portion of the column, or cyclic carbonate or a raw material rich therein may be supplied in a liquid form from an introduction port in an upper portion or an intermediate portion of the distillation column, while aliphatic monovalent alcohols or a raw material rich therein may be supplied in a gas form from an introduction port installed above a lower liquid discharge port of the distillation column and in an intermediate portion or a lower portion of the column, and this is also a preferred method.

The reaction time of the transesterification reaction that is performed in the step (I) presumably corresponds to an average residence time of a reaction liquid in the continuous multistage distillation column $T_0$. This differs depending on the shape and number of stages of the internal of the distillation column, the amount of raw materials supplied, the type and amount of a catalyst, reaction conditions, etc. and is usually 0.1 to 20 hours, preferably 0.5 to 15 hours, more preferably 1 to 10 hours.

The reaction temperature of the step (I) differs depending on the type of the raw material compound used and the type and amount of a catalyst, and is usually 30 to 300°C. Although a higher reaction temperature is preferred for enhancing a reaction rate, such a high reaction temperature easily causes side reaction. The reaction temperature is in the range of preferably 40 to 250°C, more preferably 50 to 200°C, further preferably 60 to 150°C. In the present embodiment, reactive distillation can be carried out at a column bottom temperature of 150°C or lower, preferably 130°C or lower, more preferably 110°C or lower, further preferably 100°C or lower. High reaction ratio, high selectivity, and high productivity that can be achieved even at such a low column bottom temperature is also one of the excellent features of the step (I). The reaction pressure differs depending on the type and composition of the raw material compound used, the reaction temperature, etc., and can be any of reduced pressure, normal pressure, and increased pressure. Usually, the reaction is performed in the range of 1 Pa to $2 \times 10^7$ Pa, preferably $10^3$ Pa to $10^7$ Pa, more preferably $10^4$ Pa to $5 \times 10^6$ Pa.

The reflux ratio for use in the continuous multistage distillation column $T_0$ in the step (I) is usually 0 to 10, preferably 0.01 to 5, more preferably 0.05 to 3.

The material constituting the continuous multistage distillation column $T_0$ for use in the step (I) is mainly a metal material such as carbon steel or stainless steel, and stainless steel is preferred from the viewpoint of the quality of the produced dialkyl carbonate and diols.

The production method according to the present embodiment comprises the steps of: continuously supplying dialkyl carbonate and phenol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl phenyl carbonate thus formed in a liquid form from a lower portion of the first continuous multistage distillation column to continuously produce alkyl phenyl carbonate; and continuously supplying the first column high-boiling reaction mixture into a second continuous multistage distillation column containing a catalyst, simultaneously performing a reaction

and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diphenyl carbonate thus formed in a liquid form from a lower portion of the second continuous multistage distillation column to continuously produce diphenyl carbonate (hereinafter, these two steps are also collectively referred to as a "step (II)").

Examples of the dialkyl carbonate for use in the step (II) include, but are not particularly limited to, compounds represented by the following formula described in Formula 1 described above:

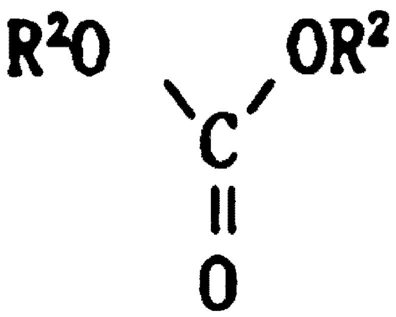

In the formula, $R^2$ is as described above.

Examples of the dialkyl carbonate having such $R^2$ include, but are not particularly limited to, dimethyl carbonate, diethyl carbonate, dipropyl carbonate (each isomer), diallyl carbonate, dibutenyl carbonate (each isomer), dibutyl carbonate (each isomer), dipentyl carbonate (each isomer), dihexyl carbonate (each isomer), diheptyl carbonate (each isomer), dioctyl carbonate (each isomer), dinonyl carbonate (each isomer), didecyl carbonate (each isomer), dicyclopentyl carbonate, dicyclohexyl carbonate, dicycloheptyl carbonate, dibenzyl carbonate, diphenethyl carbonate (each isomer), di(phenylpropyl) carbonate (each isomer), di(phenylbutyl) carbonate (each isomer), di(chlorobenzyl) carbonate (each isomer), di(methoxybenzyl) carbonate (each isomer), di(methoxymethyl) carbonate, di(methoxyethyl) carbonate (each isomer), di(chloroethyl) carbonate (each isomer), and di(cyanoethyl) carbonate (each isomer).

Among them, dialkyl carbonate wherein $R^2$ is an alkyl group having 4 or less carbon atoms and containing no halogen is preferably used in the present embodiment, and dimethyl carbonate is particularly preferred. The preferred dialkyl carbonate is further preferably dialkyl carbonate produced in a state substantially free of halogen, and is produced from, for example, alkylene carbonate substantially free of halogen and an alcohol substantially free of halogen.

The phenol for use in the step (II) is represented by the following general formula and is a compound in which a hydroxyl group is bonded directly to a phenyl group (Ph), though substituted phenol in which a phenyl group is substituted by a lower alkyl group or a lower alkoxy group may be used:

PhOH

In the present embodiment, phenol substantially free of halogen is preferably used. Thus, the diphenyl carbonate described in the present embodiment is represented by the following general formula:

PhOCOOPh

The molar ratio of the dialkyl carbonate to the phenol for use as raw materials in the step (II) is preferably 0.1 to 10. When the molar ratio falls within this range, unreacted raw materials remain in a small amount with respect to a predetermined production amount of the desired diphenyl carbonate, which are efficient. Also, energy for recovering these unreacted materials can be suppressed. In this respect, the molar ratio is more preferably 0.5 to 5, further preferably 0.8 to 3, still further preferably 1 to 2.

In the present embodiment, it is preferred to continuously produce 1 ton or more of high-purity diphenyl carbonate per hour.

Thus, in the step (II), the lowest amount of the phenol continuously supplied is usually 15Q ton/hr, preferably 13Q ton/hr, more preferably 10Q ton/hr, based on the amount (Q ton/hr) of the high-purity diphenyl carbonate to be produced. Further preferably, the lowest amount can be smaller than 8Q ton/hr.

Each of the dialkyl carbonate and the phenol for use as raw materials in the step (II) may have a high purity or may contain other compounds. The raw materials may contain, for example, compounds and reaction by-products formed in the first continuous multistage distillation column and/or the second continuous multistage distillation column. In the case of carrying out an industrial process, dialkyl carbonate and phenol that are newly introduced into the reaction

system as well as those recovered from the first continuous multistage distillation column and/or the second continuous multistage distillation column are preferably used as these raw materials. In the method of the present embodiment, a first circulation step of continuously supplying a column top component which is a second column low-boiling reaction mixture in the second continuous multistage distillation column into the first continuous multistage distillation column is performed. In this case, the second column low-boiling reaction mixture may be supplied directly into the first continuous multistage distillation column or may be supplied after a portion of the component is separated.

The concentration of alkyl aryl carbonate in the second column low-boiling reaction mixture is preferably 1% by mass or less. When the concentration of alkyl aryl carbonate in the second column low-boiling reaction mixture is 1% by mass or less, the rate of conversion of dimethyl carbonate in the first continuous multistage distillation column tends to be improved. As a result, the amounts of vapor used tend to be reduced in the first continuous multistage distillation column and the second continuous multistage distillation column because the amount of the low-boiling mixture of the second continuous multistage distillation column circulated for continuous supply into the first continuous multistage distillation column is reduced. From a similar viewpoint, the concentration of alkyl aryl carbonate in the second column low-boiling reaction mixture is preferably 0.01 to 0.5% by mass, more preferably 0.01 to 0.3% by mass.

In the present embodiment, the concentration of the alkyl aryl carbonate in the second column low-boiling reaction mixture can be measured by a method described in Examples mentioned later.

In the present embodiment to be industrially carried out, the raw materials to be supplied into the first continuous multistage distillation column preferably contain alcohols, alkyl phenyl carbonate, diphenyl carbonate, alkyl phenyl ether, and the like, and even raw materials further containing a small amount of high-boiling by-products such as Fries rearrangement products of the product phenyl carbonate or diphenyl carbonate or their derivatives are preferably used. In the present embodiment, for example, in the case of producing methyl phenyl carbonate and diphenyl carbonate by using dimethyl carbonate as the dialkyl carbonate and unsubstituted phenol as the phenol as raw materials, the raw materials preferably contain the reaction products methyl alcohol, methyl phenyl carbonate, and diphenyl carbonate and may further contain a small amount of reaction by-products anisole, phenyl salicylate, and methyl salicylate or high-boiling by-products derived therefrom.

The diphenyl carbonate obtained by the production method of the present embodiment is preferably used for producing aromatic polycarbonate through polymerization reaction with an aromatic dihydroxy compound. In this polymerization step, a large amount of phenol is formed as by-products and recovered. Use of this phenol as by-products as a raw material of the step (II) of the present embodiment is also a preferred method.

The diphenyl carbonate to be produced in the step (II) is obtained through transesterification reaction of dialkyl carbonate and phenol. This transesterification reaction includes a reaction of exchanging one or two alkoxy groups of the dialkyl carbonate with a phenoxy group of the phenol to eliminate alcohols, and a reaction of converting the formed alkyl phenyl carbonate into diphenyl carbonate and dialkyl carbonate through disproportionation reaction which is transesterification reaction between two molecules of the alkyl phenyl carbonate. In the first continuous multistage distillation column of the step (II), alkyl phenyl carbonate is mainly obtained, and in the second continuous multistage distillation column, diphenyl carbonate and dialkyl carbonate are mainly obtained through the disproportionation reaction of this alkyl phenyl carbonate. The diphenyl carbonate obtained in the step (II) is preferably totally free of halogen. Such diphenyl carbonate is useful, for example, as a raw material in industrially producing aromatic polycarbonate. This is because the presence of halogen even in an amount smaller than 1 ppm, for example, in polymerization raw materials inhibits polymerization reaction, inhibits stable production of aromatic polycarbonate, and causes reduction in the physical properties of the formed aromatic polycarbonate and stain.

The catalyst for use in the first continuous multistage distillation column and/or the second continuous multistage distillation column in the step (II) is not particularly limited and is selected from, for example, the following compounds:

A metal-containing compound is used as the catalyst, for example:

<lead compounds>

lead oxides such as PbO, $PbO_2$, and $Pb_3O_4$;
lead sulfides such as PbS and $Pb_2S$;
lead hydroxides such as $Pb(OH)_2$ and $Pb_2O_2(OH)_2$;
plumbites such as $Na_2PbO_2$, $K_2PbO_2$, $NaHPbO_2$, and $KHPbO_2$ ;
plumbates such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$;
carbonate of lead and basic salts thereof, such as $PbCO_3$ and $2PbCO_3$-$Pb(OH)_2$;
lead salts of organic acids and carbonate and basic salts thereof, such as $Pb(OCOCH_3)_2$, $Pb(OCOCH_3)_4$, and $Pb(OCOCH_3)_2 \cdot PbO \cdot 3H_2O$;
organic lead compounds such as $Bu_4Pb$, $Ph_4Pb$, $Bu_3PbCl$, $Ph_3PbBr$, $Ph_3Pb$ (or $Ph_6Pb_2$), $Bu_3PbOH$, and $Ph_3PbO$ (Bu represents a butyl group, and Ph represents a phenyl group);

alkoxyleads and aryloxyleads such as $Pb(OCH_3)_2$, $(CH_3O)Pb(OPh)$, and $Pb(OPh)_2$;
alloys of lead such as Pb-Na, Pb-Ca, Pb-Ba, Pb-Sn, and Pb-Sb; and
lead minerals such as galena and sphalerite, and hydrides of these lead compounds;

<copper family metal compounds>
salts and complexes of copper family metals such as CuCl, $CuCl_2$, CuBr, $CuBr_2$, CuI, $CuI_2$, $Cu(OAc)_2$, $Cu(acac)_2$, copper oleate, $Bu_2Cu$, $(CH_3O)_2Cu$, $AgNO_3$, AgBr, silver picrate, $AgC_6H_6ClO_4$, $[AuC{\equiv}C\text{-}C(CH_3)_3]n$, and $[Cu(C_7H_8)Cl]_4$ (acac represents an acetylacetone chelating ligand);
<alkali metal complexes>
alkali metal complexes such as Li(acac) and $LiN(C_4H_9)_2$;
<zinc complexes>
zinc complexes such as $Zn(acac)_2$;
<cadmium complexes>
cadmium complexes such as $Cd(acac)_2$;
<iron family metal compounds>
iron family metal complexes such as $Fe(C_{10}H_8)(CO)_5$, $Fe(CO)_5$, $Fe(C_4H_6)(CO)_3$, $Co(mesitylene)_2(PEt_2Ph)_2$, $CoC_5F_5(CO)_7$, Ni-π-$C_5H_5NO$, and ferrocene;
<zirconium complexes>
zirconium complexes such as $Zr(acac)_4$ and zirconocene;
<Lewis acid compounds>
Lewis acids and transition metal compounds that generate Lewis acids, such as $AlX_3$, $TiX_3$, $TiX_4$, $VOX_3$, $VX_5$, $ZnX_2$, $FeX_3$, and $SnX_4$ (wherein X is halogen, an acetoxy group, an alkoxy group, or an aryloxy group); and
<organic tin compounds>
organic tin compounds such as $(CH_3)_3SnOCOCH_3$, $(C_2H_5)_3SnOCOC_6H_5$, $Bu_3SnOCOCH_3$, $Ph_3SnOCOCH_3$, $Bu_2Sn(OCOCH_3)_2$, $Bu_2Sn(OCOC_{11}H_{23})_2$, $Ph_3SnOCH_3$, $(C_2H_5)_3SnOPh$, $Bu_2Sn(OCH_3)_2$, $Bu_2Sn(OC_2H_5)_2$, $Bu_2Sn(OPh)_2$, $Ph_2Sn(OCH_3)_2$, $(C_2H_5)_3SnOH$, $Ph_3SnOH$, $Bu_2SnO$, $(C_8H_{17})_2SnO$, $Bu_2SnCl_2$, and $BuSnO(OH)$.

The catalyst used in the present embodiment preferably contains a metal such as Pb, Cu, Zn, Fe, Co, Ni, Al, Ti, V, or Sn and is a homogeneous catalyst soluble in in the reaction system. Thus, a catalyst species in which such a metal component is bonded to an organic group is preferably used. As a matter of course, these catalytic components may be reacted with organic compounds present in the reaction system, for example, aliphatic alcohols, phenols, alkyl phenyl carbonates, diphenyl carbonates, or dialkyl carbonates, or may be heat-treated with raw materials or products prior to the reaction. The catalyst used in the present embodiment preferably has a high solubility in a reaction liquid under reaction conditions. In this respect, preferred examples of the catalyst include: PbO, $Pb(OH)_2$ and $Pb(OPh)_2$; $TiCl_4$, $Ti(OMe)_4$, $(MeO)Ti(OPh)_3$, $(MeO)_2Ti(OPh)_2$, $(MeO)_3Ti(OPh)$, and $Ti(OPh)_4$; $SnCl_4$, $Sn(OPh)_4$, $Bu_2SnO$, and $Bu_2Sn(OPh)_2$; $FeCl_3$, $Fe(OH)_3$, and $Fe(OPh)_3$; and these catalysts treated with phenol or a reaction liquid. The catalyst for use in the first continuous multistage distillation column and the catalyst for use in the second continuous multistage distillation column may be of the same type or may be of different types.

In the present embodiment, it is particularly preferred to use halogen-free raw materials and a catalyst. In this case, the produced diphenyl carbonate is totally free of halogen and is therefore useful as a raw material in industrially producing polycarbonate by a transesterification method. This is because the presence of halogen even in an amount smaller than 1 ppm, for example, in polymerization raw materials inhibits polymerization reaction, reduces the physical properties of the formed polycarbonate, and causes stain.

The first continuous multistage distillation column for use in the step (II) is preferably, for example, a continuous multistage distillation column as shown in Figure 2 that comprises a cylindrical body with a length $L_1$ (cm) and an inside diameter $D_1$ (cm), comprises a structure having an internal having the number of stages $n_1$ in the inside, and comprises a gas discharge port with an inside diameter $d_{11}$ (cm) in a column top portion or an upper portion of the column close thereto, a liquid discharge port with an inside diameter $d_{12}$ (cm) in a column bottom portion or a lower portion of the column close thereto, one or more third introduction ports in an upper portion and/or an intermediate portion of the column below the gas discharge port, and one or more fourth introduction ports in an intermediate portion and/or a lower portion of the column above the liquid discharge port, wherein $L_1$, $D_1$, $L_1/D_1$, $n_1$, $D_1/d_{11}$, and $D_1/d_{12}$ respectively satisfy the following expressions (7) to (12):

$$1500 \leq L_1 \leq 8000 \text{ ... Expression (7)}$$

$$100 \leq D_1 \leq 2000 \text{ ... Expression (8)}$$

$$2 \leq L_1/D_1 \leq 40 \text{ ... Expression (9)}$$

$$20 \leq n_1 \leq 120 \text{ ... Expression (10)}$$

$$5 \leq D_1/d_{11} \leq 30 \text{ ... Expression (11)}$$

$$3 \leq D_1/d_{12} \leq 20 \text{ ... Expression (12).}$$

The second continuous multistage distillation column for use in the step (II) is preferably, for example, a continuous multistage distillation column as shown in Figure 3 that comprises a cylindrical body with a length $L_2$ (cm) and an inside diameter $D_2$ (cm), comprises a structure having an internal portion having the number of stages $n_2$ in the inside, and comprises a gas discharge port with an inside diameter $d_{21}$ (cm) in a column top portion or an upper portion of the column close thereto, a liquid discharge port with an inside diameter $d_{22}$ (cm) in a column bottom portion or a lower portion of the column close thereto, one or more fifth introduction ports in an upper portion and/or an intermediate portion of the column below the gas discharge port, and one or more sixth introduction ports in an intermediate portion and/or a lower portion of the column above the liquid discharge port, wherein $L_2$, $D_2$, $L_2/D_2$, $n_2$, $D_2/d_{22}$, and $D_2/d_{22}$ respectively satisfy the following expressions (13) to (18):

$$1500 \leq L_2 \leq 8000 \text{ ... Expression (13)}$$

$$100 \leq D_2 \leq 2000 \text{ ... Expression (14)}$$

$$2 \leq L_2/D_2 \leq 40 \text{ ... Expression (15)}$$

$$10 \leq n_2 \leq 80 \text{ ... Expression (16)}$$

$$2 \leq D_2/d_{21} \leq 15 \text{ ... Expression (17)}$$

$$5 \leq D_2/d_{22} \leq 30 \text{ ... Expression (18).}$$

By using the first continuous multistage distillation column and the second continuous multistage distillation column that simultaneously satisfy the expressions (7) to (18), diphenyl carbonate can be stably produced from dialkyl carbonate and phenol with high selectivity and high productivity at an industrial scale of approximately 0.85 tons or more, preferably 1 ton or more, per hour for a long period of, for example, 2000 hours or longer, preferably 3000 hours or longer, more preferably 5000 hours or longer. The reason is not clear why the method of the present embodiment allows for the production of aromatic carbonate having such excellent effects at an industrial scale. The effects are presumably combined effects brought about by a combination of the conditions of the expressions (7) to (18). Preferred ranges of the respective factors constituting the continuous multistage distillation columns for use in the step (II) will be given below.

When each of $L_1$ (cm) and $L_2$ (cm) is 1500 or more, the desired production amount can be achieved because reaction ratio is improved. For reducing the cost of equipment while securing the reaction ratio that can achieve the desired production amount, each of $L_1$ and $L_2$ is preferably 8000 or less. $L_1$ (cm) and $L_2$ (cm) are more preferably in the ranges of $2000 \leq L_1 \leq 6000$ and $2000 \leq L_2 \leq 6000$, respectively, further preferably $2500 \leq L_1 \leq 5000$ and $2500 \leq L_2 \leq 5000$, respectively.

When each of $D_1$ (cm) and $D_2$ (cm) is 100 or more, the desired production amount can be achieved. For reducing the cost of equipment while achieving the desired production amount, each of $D_2$ and $D_2$ is preferably 2000 or less. $D_1$ (cm) and $D_2$ (cm) are more preferably in the ranges of $150 \leq D_1 \leq 1000$ and $150 \leq D_2 \leq 1000$, respectively, further preferably $200 \leq D_1 \leq 800$ and $200 \leq D_2 \leq 800$, respectively.

The first continuous multistage distillation column and the second continuous multistage distillation column

may have the same inside diameter from the respective upper to lower portions of the columns or may have partially different inside diameters as long as $D_1$ and $D_2$ fall within the ranges described above. In these continuous multistage distillation columns, for example, the upper inside diameters of the columns may be smaller or larger than the lower inside diameters of the columns.

When each of $L_1/D_1$ and $L_2/D_2$ is 2 or more or is 40 or less, stable operation is easy to perform. Particularly, when each of the ratios is 40 or less, long-term stable operation is easy to perform because difference in pressure between the upper and lower portions of the column can be prevented from becoming too large. Besides, side reaction can be suppressed, and selectivity tends to be improved, because a temperature in the lower portion of the column can be decreased. $L_1/D_1$ and $L_2/D_2$ are more preferably in the ranges of $3 \leq L_1/D_1 \leq 30$ and $3 \leq L_2/D_2 \leq 30$, respectively, further preferably $5 \leq L_1/D_1 \leq 15$ and $5 \leq L_2/D_2 \leq 15$, respectively.

When $n_1$ is 20 or more, the desired production amount in the first continuous multistage distillation column can be achieved because reaction ratio is improved. For reducing the cost of equipment while securing the reaction ratio that can achieve the desired production amount, $n_1$ is preferably 120 or less. When $n_1$ is 120 or less, long-term stable operation of the first continuous multistage distillation column is easy to perform because difference in pressure between the upper and lower portions of the column can be prevented from becoming too large. Besides, side reaction can be suppressed, and selectivity tends to be improved, because a temperature in the lower portion of the column can be decreased. $n_1$ is more preferably in the range of $30 \leq n_1 \leq 100$, further preferably $40 \leq n_1 \leq 90$.

When $n_2$ is 10 or more, the desired production amount in the second continuous multistage distillation column can be achieved because reaction ratio is improved. For reducing the cost of equipment while securing the reaction ratio that can achieve the desired production amount, $n_2$ is preferably 80 or less. When $n_2$ is 80 or less, long-term stable operation of the second continuous multistage distillation column is easy to perform because difference in pressure between the upper and lower portions of the column can be prevented from becoming too large. Besides, side reaction can be suppressed, and selectivity tends to be improved, because a temperature in the lower portion of the column can be decreased. $n_2$ is more preferably in the range of $15 \leq n_2 \leq 60$, further preferably $20 \leq n_2 \leq 50$.

When $D_1/d_{11}$ is 5 or more, the cost of equipment of the first continuous multistage distillation column can be reduced. Stable operation of the first continuous multistage distillation column is easy to perform because a gas component can be prevented from coming out of the system. When the ratio is 30 or less, the amount of a gas component discharged is relatively large and stable operation is easy to perform. Besides, reaction ratio tends to be improved. $D_1/d_{11}$ is more preferably in the range of $8 \leq D_1/d_{11} \leq 25$, further preferably $10 \leq D_1/d_{11} \leq 20$. When $D_2/d_{21}$ is 2 or more, the cost of equipment of the second continuous multistage distillation column can be reduced. Stable operation of the second continuous multistage distillation column is easy to perform because a gas component can be prevented from coming out of the system. When the ratio is 15 or less, the amount of a gas component discharged is relatively large and stable operation is easy to perform. Besides, reaction ratio tends to be improved. $D_2/d_{21}$ is more preferably in the range of $5 \leq D_2/d_{21} \leq 12$, further preferably $3 \leq D_2/d_{21} \leq 10$.

When $D_1/d_{12}$ is 3 or more, the cost of equipment of the first continuous multistage distillation column can be reduced. The amount of a liquid discharged is relatively small, and stable operation of the first continuous multistage distillation column is easy to perform. When the ratio is 20 or less, a flow rate in a liquid discharge port or a piping can be prevented from being rapidly accelerated, erosion is unlikely to occur, and the corrosion of an apparatus can be suppressed. $D_1/d_{12}$ is more preferably in the range of $5 \leq D_1/d_{12} \leq 18$, further preferably $7 \leq D_1/d_{12} \leq 15$. When $D_2/d_{22}$ is 5 or more, the cost of equipment of the second continuous multistage distillation column can be reduced. The amount of a liquid discharged is relatively small, and stable operation of the second continuous multistage distillation column is easy to perform. When the ratio is 30 or less, a flow rate in a liquid discharge port or a piping can be prevented from being rapidly accelerated, erosion is unlikely to occur, and the corrosion of an apparatus can be suppressed. $D_2/d_{22}$ is more preferably in the range of $7 \leq D_2/d_{22} \leq 25$, further preferably $9 \leq D_2/d_{22} < 20$.

In the step (II), the factors $d_{11}$ and $d_{12}$ further preferably satisfy the expression (29), and the factors $d_{21}$ and $d_{22}$ further preferably satisfy the expression (30) :

$$1 \leq d_{12}/d_{11} \leq 5 \ \ldots \ \text{Expression (29)}$$

$$1 \leq d_{21}/d_{22} \leq 6 \ \ldots \ \text{Expression (30)}.$$

The long-term stable operation described in the step (II) means that operation can be continued for 1000 hours or longer, preferably 3000 hours or longer, further preferably 5000 hours or longer, in a steady state based on operation conditions without flooding, weeping, piping clogging or erosion, or the like, and a predetermined amount of diphenyl carbonate is produced while high selectivity is maintained.

In the step (II), diphenyl carbonate is stably produced with high selectivity for a long period at high productivity

of preferably 1 ton or more per hour, and more preferably 2 tons or more, further preferably 3 tons or more, of diphenyl carbonate per hour are produced. In the step (II), when $L_1$, $D_1$, $L_1/D_1$, $n_1$, $D_1/d_{11}$, and $D_1/d_{12}$ of the first continuous multistage distillation column satisfy $2000 \leq L_1 \leq 6000$, $150 \leq D_1 \leq 1000$, $3 \leq L_1/D_1 \leq 30$, $30 \leq n_1 \leq 100$, $8 \leq D_1/d_{11} \leq 25$, and $5 \leq D_1/d_{12} \leq 18$, respectively, and $L_2$, $D_2$, $L_2/D_2$, $n_2$, $D_2/d_{21}$, and $D_2/d_{22}$ of the second continuous multistage distillation column satisfy $2000 \leq L_2 \leq 6000$, $150 \leq D_2 \leq 1000$, $3 \leq L_2/D_2 \leq 30$, $15 \leq n_2 \leq 60$, $2.5 \leq D_2/d_{21} \leq 12$, and $7 \leq D_2/d_{22} \leq 25$, respectively, 2 tons or more, preferably 2.5 tons or more, further preferably 3 tons or more, of diphenyl carbonate per hour are produced.

In the step (II), when $L_1$, $D_1$, $L_1/D_1$, $n_1$, $D_1/d_{11}$, and $D_1/d_{12}$ of the first continuous multistage distillation column satisfy $2500 \leq L_1 \leq 5000$, $200 \leq D_1 \leq 800$, $5 \leq L_1/D_1 \leq 15$, $40 \leq n_1 \leq 90$, $10 \leq D_1/d_{11} \leq 25$, and $7 \leq D_1/d_{12} \leq 15$, respectively, and $L_2$, $D_2$, $L_2/D_2$, $n_2$, $D_2/d_{21}$, and $D_2/d_{22}$ of the second continuous multistage distillation column satisfy $2500 \leq L_2 \leq 5000$, $200 \leq D_2 \leq 800$, $5 \leq L_2/D_2 \leq 10$, $20 \leq n_2 \leq 50$, $3 \leq D_2/d_{21} \leq 10$, and $9 \leq D_2/d_{22} \leq 20$, respectively, 3 tons or more, preferably 3.5 tons or more, further preferably 4 tons or more, of diphenyl carbonate per hour are produced.

The selectivity of diphenyl carbonate described in the step (II) is based on the reacted phenol. In the step (II), high selectivity of usually 95% or more, preferably high selectivity of 97% or more, more preferably 99% or more, can be achieved.

Each of the first continuous multistage distillation column and the second continuous multistage distillation column for use in the step (II) is preferably a distillation column having a tray and/or a packing as the internal. The internal described in the present embodiment means a portion where the contact between a gas and a liquid is actually performed in each distillation column. Such a tray is preferably the tray described in the section of the step (I). The "number of stages of the internal" is as described above.

In the first continuous multistage distillation column of the step (II), a reaction of forming alkyl phenyl carbonate from dialkyl carbonate and phenol is mainly performed. This reaction has a very small equilibrium constant and furthermore, has a slow reaction rate. Therefore, the first continuous multistage distillation column for use in reactive distillation is more preferably a distillation column of plate column type having a tray in the internal. In the second continuous multistage distillation column, a reaction of disproportioning the alkyl phenyl carbonate is mainly performed. This reaction also has a very small equilibrium constant and furthermore, has a slow reaction rate. However, the second continuous multistage distillation column for use in reactive distillation is more preferably a distillation column having both a packing and a tray in the internal. In the second continuous multistage distillation column, the packing is preferably installed in an upper portion, and the tray is preferably installed in a lower portion. The packing of the second continuous multistage distillation column is preferably a structured packing, and the structured packing is particularly preferably Mellapak.

The tray to be installed in each of the first continuous multistage distillation column and the second continuous multistage distillation column is a sieve tray having a sieve part and a downcomer part because of an excellent relationship between functions and the cost of equipment in particular. The sieve tray preferably has 100 to 1000 holes per $m^2$ of the area of the sieve part. The number of holes is more preferably 120 to 900, further preferably 150 to 800, per $m^2$ of the area.

The cross section per hole of the sieve tray is preferably 0.5 to 5 $cm^2$. The cross section per hole is more preferably 0.7 to 4 $cm^2$, further preferably 0.9 to 3 $cm^2$. The case is particularly preferred where the sieve tray has 100 to 1000 holes per $m^2$ of the area of the sieve part, and the cross section per hole is 0.5 to 5 $cm^2$.

The step (II), when carried out, comprises: continuously supplying raw materials dialkyl carbonate and phenol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl phenyl carbonate thus formed in a liquid form from a lower portion of the first continuous multistage distillation column; and continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diphenyl carbonate thus formed in a liquid form from a lower portion of the second continuous multistage distillation column to continuously produce diphenyl carbonate.

The raw materials may contain reaction products alcohols, alkyl phenyl carbonate, diphenyl carbonate, and alkyl phenyl ether or reaction by-products such as high-boiling compounds, as described above. In the present embodiment to be actually industrially carried out, the raw materials preferably contain a small amount of these compounds, in consideration of equipment and cost required for separation and purification in other steps.

A column top component of the first continuous multistage distillation column in the step (II) often contains dialkyl carbonate, phenol, alkyl aryl ether, and the like in addition to alcohols formed through the reaction and may further contain a small amount of alkyl aryl carbonate, diphenyl carbonate, and the like. This column top component of the first continuous multistage distillation column may be used directly as aliphatic monovalent alcohols in the step (I) and is

preferably used as raw materials in the step (I) after the content of substances having a higher boiling point than that of the alcohols is decreased by distillation or the like. A mixture of the alcohols and dialkyl carbonate is particularly preferably used as aliphatic monovalent alcohols in the step (I).

In the step (II), the raw materials dialkyl carbonate and phenol, when continuously supplied into the first continuous multistage distillation column, may be supplied in a liquid form and/or in a gas form from an introduction port installed at one or several locations below an upper gas discharge port of the first distillation column and in an upper portion or an intermediate portion of the column, or a raw material rich in phenol may be supplied in a liquid form from an introduction port in an upper portion of the first distillation column, while a raw material rich in dialkyl carbonate may be supplied in a gas form from an introduction port installed above a lower liquid discharge port of the first distillation column and in a lower portion of the column, and this is also a preferred method.

In the step (II), the first column high-boiling reaction mixture containing alkyl phenyl carbonate continuously discharged from a lower portion of the first continuous multistage distillation column is continuously supplied into the second continuous multistage distillation column. As for a supply position thereof, the reaction mixture is preferably supplied in a liquid form and/or in a gas form from an introduction port installed at one or several locations below an upper gas discharge port of the second continuous multistage distillation column and in an upper portion or an intermediate portion of the column. In a preferred embodiment of the present embodiment, in the case of using a distillation column having a packing portion in an upper portion and a tray portion in a lower portion as the second continuous multistage distillation column, at least one introduction port is preferably installed between the packing portion and the tray portion. When the packing is formed from a plurality of (two or more) structured packings, an introduction port may be installed in space flanked by these structured packings, and this is also a preferred method.

In the step (II), reflux operation of condensing a gas component discharged from the column top of each of the first continuous multistage distillation column and the second continuous multistage distillation column, and then returning a portion of the condensate to the upper portion of each of the distillation columns is also a preferred method. In this case, the reflux ratio of the first continuous multistage distillation column is 0 to 10, and the reflux ratio of the second continuous multistage distillation column is in the range of 0.01 to 10, preferably 0.08 to 5, more preferably 0.1 to 2. In a preferred embodiment, the reflux ratio of the first continuous multistage distillation column is 0 which means that no reflux operation is performed.

In the step (II), the method for allowing the first continuous multistage distillation column to contain a homogeneous catalyst can be any method. The homogeneous catalyst is preferably supplied into the first continuous multistage distillation column from a position above the intermediate portion of the distillation column. Also, the catalyst is preferably supplied such that a reaction liquid comes into contact with the catalyst in a region of at least seven or more plates, preferably ten or more plates, more preferably 15 or more plates, of the first continuous multistage distillation column. In this case, a solution of the catalyst dissolved in raw materials or a reaction liquid may be introduced together with the raw materials, or this catalyst solution may be introduced from an introduction port different from that for the raw materials. In the present embodiment, the amount of the catalyst used in the first continuous multistage distillation column differs depending on difference in the type of the catalyst used, the types and quantitative ratio of the raw materials, and reaction conditions such as a reaction temperature and a reaction pressure. The catalyst is used at usually 0.0001 to 30% by mass, preferably 0.005 to 10% by mass, more preferably 0.001 to 1% by mass, in terms of a ratio to the total mass of the raw materials.

In the step (II), the method for allowing the second continuous multistage distillation column to contain a homogeneous catalyst can be any method. The homogeneous catalyst is preferably supplied into the second continuous multistage distillation column from a position above the intermediate portion of the distillation column. In this case, a solution of the catalyst dissolved in raw materials or a reaction liquid may be introduced together with the raw materials, or this catalyst solution may be introduced from an introduction port different from that for the raw materials. In the present embodiment, the amount of the catalyst used in the second continuous multistage distillation column differs depending on difference in the type of the catalyst used, the types and quantitative ratio of the raw materials, and reaction conditions such as a reaction temperature and a reaction pressure. The catalyst is used at usually 0.0001 to 30% by mass, preferably 0.005 to 10% by mass, more preferably 0.001 to 1% by mass, in terms of a ratio to the total mass of the raw materials.

In the step (II), the catalyst for use in the first continuous multistage distillation column and the catalyst for use in the second continuous multistage distillation column may be of the same type or may be of different types. Preferably, the same type of catalyst is used. The catalyst is further preferably of the same type and can be dissolved in both reaction liquids. This embodiment is preferred because the catalyst is usually discharged, in a state dissolved in the high-boiling reaction mixture of the first continuous multistage distillation column, together with alkyl phenyl carbonate and the like from a lower portion of the first distillation column, and supplied directly into the second continuous multistage distillation column. If necessary, the catalyst may be newly added to the second continuous multistage distillation column.

The reaction time of the transesterification reaction to be performed in the step (II) presumably corresponds to an average residence time of a reaction liquid in each of the first continuous multistage distillation column and the second

continuous multistage distillation column. This reaction time differs depending on the shape and number of stages of the internal of each of the distillation columns, the amount of raw materials supplied, the type and amount of a catalyst, reaction conditions, etc. and is usually 0.01 to 10 hours, preferably 0.05 to 5 hours, more preferably 0.1 to 3 hours, in each of the first continuous multistage distillation column and the second continuous multistage distillation column.

The reaction temperature of the first continuous multistage distillation column differs depending on the type of the raw material compound used and the type and amount of a catalyst, and is usually in the range of 100 to 350°C. A higher reaction temperature is preferred for enhancing a reaction rate and however, is not preferred because such a high reaction temperature easily causes side reaction and increases by-products such as alkyl phenyl ether. In this respect, the reaction temperature of the first continuous multistage distillation column is in the range of preferably 130 to 280°C, more preferably 150 to 260°C, further preferably 180 to 250°C.

The reaction temperature of the second continuous multistage distillation column differs depending on the type of the raw material compound used and the type and amount of a catalyst, and is usually in the range of 100 to 350°C. A higher reaction temperature is preferred for enhancing a reaction rate and however, is not preferred because such a high reaction temperature easily causes side reaction and increases by-products such as alkyl phenyl ether, or Fries rearrangement reaction products of raw materials or the product alkyl phenyl carbonate or diphenyl carbonate or their derivatives. In this respect, the reaction temperature of the second continuous multistage distillation column is in the range of preferably 130 to 280°C, more preferably 150 to 260°C, further preferably 180 to 250°C.

The reaction pressure of the first continuous multistage distillation column differs depending on the type and composition of the raw material compound used, the reaction temperature, etc., and can be any of reduced pressure, normal pressure, and increased pressure in the first continuous multistage distillation column. Usually, the column top pressure is in the range of 0.1 Pa to $2 \times 10^7$ Pa, preferably $10^5$ Pa to $10^7$ Pa, more preferably $2 \times 10^5$ Pa to $5 \times 10^6$ Pa.

The reaction pressure of the second continuous multistage distillation column differs depending on the type and composition of the raw material compound used, the reaction temperature, etc., and can be any of reduced pressure, normal pressure, and increased pressure. Usually, the column top pressure is in the range of 0.1 Pa to $2 \times 10^7$ Pa, preferably $10^3$ Pa to $10^6$ Pa, more preferably $5 \times 10^3$ Pa to $10^5$ Pa.

Two or more distillation columns may be used as the first continuous multistage distillation column in the step (II). In this case, the two or more distillation columns may be connected in series, may be connected in parallel, or may be connected with in series and in parallel in combination. Two or more distillation columns may be used as the second continuous multistage distillation column in the step (II). In this case, the two or more distillation columns may be connected in series, may be connected in parallel, or may be connected with in series and in parallel in combination.

The material constituting the first continuous multistage distillation column and the second continuous multistage distillation column for use in the step (II) is mainly a metal material such as carbon steel or stainless steel, and stainless steel is preferred from the viewpoint of the quality of the produced aromatic carbonate.

A material that is composed mainly of Fe and contains 2% by mass or more of Mo and 18% by mass or less of Cr is preferably used in a liquid-contacting part in a lower portion of the second continuous multistage distillation column. Use of such a material in a liquid-contacting part in a lower portion of the second continuous multistage distillation column tends to be able to suppress the corrosion of the distillation column even if the concentration of a high-boiling substance in a component is elevated in the lower portion of the second continuous multistage distillation column. The composition of the material in the liquid-contacting part in the lower portion of the second continuous multistage distillation column is more preferably 60 to 72% by mass of Fe, 2 to 3% by mass of Mo, and 16 to 18% by mass of Cr.

In the present embodiment, the term "composed mainly of" means 40% by mass or more and is preferably 50% by mass or more, more preferably 60% by mass.

The production method of the present embodiment comprises: a first purification step of continuously introducing the second column high-boiling reaction mixture containing diphenyl carbonate to a high-boiling substance separation column A, and continuously separating by distillation the mixture into a column top component ($A_T$) containing diphenyl carbonate and a column bottom component ($A_B$) containing the catalyst; and a second purification step of continuously introducing the column top component ($A_T$) to a diphenyl carbonate purifying column B having a side cut discharge port, and continuously separating by distillation the component into three components, a column top component ($B_T$), a side cut component ($B_S$), and a column bottom component ($B_B$), thereby obtaining diphenyl carbonate as the side cut component ($B_S$) (hereinafter, the first and second purification steps are also collectively referred to as a "step (III) ") .

The second column high-boiling reaction mixture continuously discharged in a liquid form from a lower portion of the second continuous multistage distillation column in the step (II) is composed mainly of diphenyl carbonate and also usually contains unreacted alkyl phenyl carbonate, a small amount of unreacted raw materials, a small amount of high-boiling by-products, and a catalytic component. Thus, the purification step (III) is carried out from the viewpoint of obtaining high-purity diphenyl carbonate from the second column high-boiling reaction mixture. The step (III) employs two distillation columns (a high-boiling substance separation column A and a diphenyl carbonate purifying column B having a side cut discharge port), and can involve: continuously separating the mixture into a column top component ($A_T$) composed mainly of unreacted alkyl phenyl carbonate, a small amount of unreacted raw materials, and diphenyl

carbonate, and a column bottom component ($A_B$) composed mainly of a small amount of high-boiling by-products, etc. and/or a catalytic component in the high-boiling substance separation column A; and continuously supplying the column top component ($A_T$) of the high-boiling substance separation column into the diphenyl carbonate purifying column B, and continuously separating the component into three components, a column top component ($B_T$), a side cut component ($B_S$), and a column bottom component ($B_B$), in the diphenyl carbonate purifying column B to obtain high-purity diphenyl carbonate as the side cut component ($B_S$).

In the step (III), the high-boiling substance separation column A is preferably, for example, a continuous multistage distillation column that comprises a cylindrical body with a length $L_A$ (cm) and an inside diameter $D_A$ (cm) and comprises an internal having the number of stages $n_A$ in the inside, wherein $L_A$, $D_A$, and $n_A$ respectively satisfy the following expressions (19) to (21) :

$$800 \leq L_A \leq 3000 \ldots \text{Expression (19)}$$

$$100 \leq D_A \leq 1000 \ldots \text{Expression (20)}$$

$$20 \leq n_A \leq 100 \ldots \text{Expression (21).}$$

The diphenyl carbonate purifying column B is preferably, for example, a continuous multistage distillation column that comprises a cylindrical body with a length $L_B$ (cm) and an inside diameter $D_B$ (cm), comprises a structure having an internal in the inside, and comprises an introduction port $B_1$ in an intermediate portion of the column and a side cut discharge port $B_2$ between the introduction port $B_1$ and the column bottom, wherein the number of stages of the internal from the introduction port $B_1$ through an upper portion is defined as $n_{B1}$, the number of stages of the internal between the introduction port $B_1$ and the side cut discharge port $B_2$ is defined as $n_{B2}$, the number of stages of the internal from the side cut discharge port $B_2$ through a lower portion is defined as $n_{B3}$, and the total number of stages ($n_{B1} + n_{B2} + n_{B3}$) is defined as $n_B$, wherein $L_B$, $D_B$, $n_{B1}$, $n_{B2}$, $n_{B3}$, and $n_B$ respectively satisfy the following expressions (22) to (27):

$$1000 \leq L_B \leq 5000 \ldots \text{Expression (22)}$$

$$100 \leq D_B \leq 1000 \ldots \text{Expression (23)}$$

$$5 \leq n_{B1} \leq 20 \ldots \text{Expression (24)}$$

$$12 \leq n_{B2} \leq 40 \ldots \text{Expression (25)}$$

$$3 \leq n_{B3} \leq 15 \ldots \text{Expression (26)}$$

$$20 \leq n_B \leq 70 \ldots \text{Expression (27).}$$

All of these conditions are simultaneously satisfied, whereby high-purity diphenyl carbonate tends to be able to be stably produced from the second column high-boiling reaction mixture containing diphenyl carbonate obtained in the step (II) at an industrial scale of 1 ton or more per hour for a long period of, for example, 2000 hours or longer, preferably 3000 hours or longer, further preferably 5000 hours or longer. The reason is not clear why the method of the present embodiment allows for the production of high-purity diphenyl carbonate at an industrial scale having such excellent effects. The effects are presumably brought about by a combination of the high-boiling substance separation column A and the diphenyl carbonate purifying column B which satisfy the conditions of the expressions (19) to (27). Preferred ranges of the respective factors will be given below.

When $L_A$ (cm) is 800 or more, separation efficiency tends to be improved because the internal that can be installed in the inside of the high-boiling substance separation column A has a height sufficient therefor. For reducing the cost of equipment while achieving the desired separation efficiency, $L_A$ is preferably 3000 or less. $L_A$ (cm) is more

preferably in the range of $1000 \leq L_A \leq 2500$, further preferably $1200 \leq L_A \leq 2000$.

When $D_A$ (cm) is 100 or more, the desired production amount can be achieved. For reducing the cost of equipment while achieving the desired production amount, $D_A$ is preferably 1000 or less. $D_A$ (cm) is more preferably in the range of $200 \leq D_A \leq 600$, further preferably $250 \leq D_A \leq 450$.

When $n_A$ is 20 or more, the desired high purity can be achieved because separation efficiency is improved. For reducing the cost of equipment while achieving the desired separation efficiency, $n_A$ is preferably 100 or less. When $n_A$ is 100 or less, long-term stable operation of the high-boiling substance separation column A is easy to perform because difference in pressure between the upper and lower portions of the column can be prevented from becoming too large. Besides, side reaction tends to be suppressed because a temperature in the lower portion of the column can be decreased. $n_A$ is more preferably in the range of $30 \leq n_A \leq 70$, further preferably $35 \leq n_A \leq 60$.

When $L_B$ (cm) is 1000 or more, separation efficiency tends to be improved because the internal that can be installed in the inside of the diphenyl carbonate purifying column B has a height sufficient therefor. For reducing the cost of equipment while achieving the desired separation efficiency, $L_B$ is preferably 5000 or less. $L_B$ (cm) is more preferably in the range of $1500 \leq L_B \leq 3000$, further preferably $1700 \leq L_B \leq 2500$.

When $D_B$ (cm) is 100 or more, the desired production amount can be achieved. For reducing the cost of equipment while achieving the desired production amount, $D_B$ is preferably 1000 or less. $D_B$ (cm) is more preferably in the range of $150 \leq D_B \leq 500$, further preferably $200 \leq D_B \leq 400$.

When $n_B$ is 20 or more, the desired high purity can be achieved because separation efficiency as the whole column is improved. For reducing the cost of equipment while achieving the desired separation efficiency, $n_B$ is preferably 70 or less. When $n_B$ is 70 or less, long-term stable operation of the diphenyl carbonate purifying column B is easy to perform because difference in pressure between the upper and lower portions of the column can be prevented from becoming large. Besides, side reaction tends to be suppressed because a temperature in the lower portion of the column can be decreased. $n_B$ is more preferably in the range of $25 \leq n_B \leq 55$, further preferably $30 \leq n_B \leq 50$. For stably obtaining the desired high-purity diphenyl carbonate for a long time, $n_{B1}$, $n_{B2}$, and $n_{B3}$ are preferably in the range of $5 \leq n_{B1} \leq 20$, $12 \leq n_{B2} \leq 40$, and $3 \leq n_{B3} \leq 15$, respectively, more preferably in the range of $7 \leq n_{B1} \leq 15$, $12 \leq n_{B2} \leq 30$, and $3 \leq n_{B3} \leq 10$, respectively.

In carrying out the present embodiment, the high-boiling substance separation column A is preferably operated at a column bottom temperature ($T_A$) of 185 to 280°C and a column top pressure ($P_A$) of 1000 to 20000 Pa, and the diphenyl carbonate purifying column B is preferably operated at a column bottom temperature ($T_B$) of 185 to 280°C and a column top pressure ($P_B$) of 1000 to 20000 Pa.

When $T_A$ is 185°C or higher, equipment that retains high vacuum is not necessary because the column top pressure does not have to be lower. Also, equipment can be downsized. When $T_A$ is 280°C or lower, the formation of high-boiling by-products during distillation tends to be able to be suppressed. $T_A$ is in the range of more preferably 190 to 240°C, further preferably 195 to 230°C.

When $P_A$ is 1000 Pa or higher, large equipment is not necessary for retaining high vacuum. When $P_A$ is 20000 Pa or lower, by-products tend to be able to be suppressed because of a low distillation temperature. $P_A$ is in the range of more preferably 2000 to 15000 Pa, further preferably 3000 to 13000 Pa.

When $T_B$ is 185°C or higher, equipment that retains high vacuum is not necessary because the column top pressure does not have to be lower. Also, equipment can be downsized. When $T_B$ is 280°C or lower, the formation of high-boiling by-products during distillation tends to be able to be suppressed. $T_B$ is in the range of more preferably 190 to 240°C, further preferably 195 to 230°C.

When $P_B$ is 1000 Pa or higher, large equipment is not necessary for retaining high vacuum. When $P_B$ is 20000 Pa or lower, by-products tend to be able to be suppressed because of a low distillation temperature. $P_B$ is in the range of more preferably 2000 to 15000 Pa, further preferably 3000 to 13000 Pa.

The high-boiling substance separation column A and the diphenyl carbonate purifying column B may have the same inside diameter from the respective upper to lower portions of the columns or may have partially different inside diameters as long as $D_A$ and $D_B$ fall within the ranges described above. In these continuous multistage distillation columns, for example, the upper inside diameters of the columns may be smaller or larger than the lower inside diameters of the columns.

Each of the high-boiling substance separation column A and the diphenyl carbonate purifying column B for use in the step (III) is preferably a distillation column having a tray and/or a packing as the internal. The internal described in the present embodiment means a portion where the contact between a gas and a liquid is actually performed in the distillation column. Such a tray is preferably the tray described in the section of the step (I). The "number of stages of the internal" is as described above.

The high-boiling substance separation column A in the step (III) preferably comprises a packing as the internal and preferably comprises a structured packing as the packing. The diphenyl carbonate purifying column B preferably comprises a packing as the internal and preferably comprises one or two or more structured packings.

The high-boiling reaction mixture continuously discharged from the column bottom of the second continuous

multistage distillation column in the step (II) contains 0.05 to 2% by mass of dialkyl carbonate, 1 to 20% by mass of phenol, 0.05 to 2% by mass of alkyl phenyl ether, 10 to 45% by mass of alkyl phenyl carbonate, 50 to 80% by mass of diphenyl carbonate, 0.1 to 5% by mass of high-boiling by-products, and 0.001 to 5% by mass of the catalyst. Therefore, it is preferred to continuously supply this continuously discharged column bottom liquid directly to the high-boiling substance separation column A in the step (III).

The composition of the reaction mixture varies depending on conditions of the transesterification reaction of dialkyl carbonate and phenol, the type and amount of the catalyst, etc. A reaction mixture having almost constant composition can be produced as long as the transesterification reaction is performed under constant conditions. Therefore, the composition of the reaction mixture to be supplied to the high-boiling substance separation column A is almost constant. However, in the step (III), the reaction mixture can be separated with almost the same separation efficiency even if its composition varies within the range described above. This is one of the features of the step (III) used in the present embodiment.

In the step (III), a column bottom liquid of the second continuous multistage distillation column of the step (II), when continuously supplied into the high-boiling substance separation column A, may be supplied in a liquid form from an introduction port installed at one or several locations below the intermediate portion of the separation column A, or supply into the separation column A through a reboiler from a piping disposed in the lower portion of the reboiler of the separation column A is also a preferred method. The amount of the column bottom liquid of the second continuous multistage distillation column supplied into the high-boiling substance separation column A varies depending on the production amount of the high-purity diphenyl carbonate to be produced, the concentration of the diphenyl carbonate in the reaction mixture, separation conditions for the separation column A, etc., and is usually approximately 2 ton/hr or more, preferably approximately 6 ton/hr or more, further preferably approximately 10 ton/hr or more.

The high-boiling reaction mixture of the second continuous multistage distillation column continuously supplied into the high-boiling substance separation column A is separated into a column top component ($A_T$) having a large proportion of diphenyl carbonate and a large proportion of compounds having a lower boiling point than that of diphenyl carbonate, such as unreacted raw materials, alkyl phenyl ether, and alkyl phenyl carbonate, and a column bottom component ($A_B$) containing a small amount of diphenyl carbonate, the catalyst, and high-boiling by-products. The column bottom component ($A_B$) may contain a small amount of alkyl phenyl carbonate. These organic materials in the column bottom component help to dissolve a catalytic component and maintain a liquid form. Usually, the whole amount or a portion of this column bottom component ($A_B$) is usually continuously suppled as a catalytic component for transesterification reaction directly into the first continuous multistage distillation column and/or into the second continuous multistage distillation column of the step (II), and circulated and reused. In some cases, this component is separated from organic materials in a catalyst recovery step, then regenerated as a catalyst, and circulated and reused.

In the step (III), for example, by-products having a higher boiling point than that of diphenyl carbonate, such as phenyl salicylate, xanthone, phenyl methoxybenzoate, and 1-phenoxycarbonyl-2-phenoxycarboxy-phenylene, and a catalytic component are almost completely separated as the column bottom component ($A_B$) in this high-boiling substance separation column A, and the content thereof in the column top component ($A_T$) can be easily set to usually 200 ppm or less, preferably 100 ppm or less, more preferably 50 ppm or less. This is one of the features of the step (III). The column top component ($A_T$) rarely contains these high-boiling by-products, and furthermore, a large proportion of diphenyl carbonate in the introduced reaction mixture can be discharged from the column top. This is also one of the features of the step (III). In the step (III), 95% or more, preferably 96% or more, more preferably 98% or more, of diphenyl carbonate in the reaction mixture continuously supplied into the high-boiling substance separation column A can be discharged from the column top. In the step (III), usually 90 to 97% by mass of the continuously supplied liquid is continuously discharged as the column top component ($A_T$) from the column top, and 10 to 3% by mass thereof is continuously discharged as the column bottom component ($A_B$) from the column bottom, though depending on the composition of the high-boiling reaction mixture of the second continuous multistage distillation column supplied to the separation column A. The composition of the column top component ($A_T$) is usually 0.05 to 1% by mass of dialkyl carbonate, 1 to 10% by mass of phenol, 0.05 to 0.5% by mass of alkyl phenyl ether, 20 to 40% by mass of alkyl phenyl carbonate, and 50 to 80% by mass of diphenyl carbonate, and the content of high-boiling by-products is usually 200 ppm or less, preferably 100 ppm or less, more preferably 50 ppm.

In the step (III), the reflux ratio of the high-boiling substance separation column A is in the range of 0.01 to 10, preferably 0.08 to 5, more preferably 0.1 to 3.

The amount of the column top component ($A_T$) continuously discharged from the column top of the high-boiling substance separation column A is usually approximately 90 to 97% of the high-boiling reaction mixture of the second continuous multistage distillation column supplied into the separation column A, as described above. This amount is continuously supplied directly into the purifying column B from the introduction port B1 disposed in the intermediate portion of the diphenyl carbonate purifying column B, and continuously separated into three components, a column top component ($B_T$), a side cut component ($B_S$), and a column bottom component ($B_B$). All of components having a lower boiling point than that of diphenyl carbonate contained in the column top component ($A_T$) of the separation column A

supplied into the purifying column B are continuously discharged as the column top component ($B_T$) from the column top, while a small amount of a liquid is continuously discharged from the column bottom. The column top component ($B_T$) contains a small amount of diphenyl carbonate, the amount of which is usually 1 to 9%, preferably 3 to 8%, based on the supplied diphenyl carbonate. The diphenyl carbonate in this column top component ($B_T$) is separated by another distillation column for separating the column top component ($B_T$) and recovered. Such separation of the diaryl carbonate as a column bottom component of another distillation column and its recovery by return into the high-boiling substance separation column A and/or the diphenyl carbonate purifying column B is also a preferred method.

The column bottom component ($B_B$) contains diphenyl carbonate and a small amount of high-boiling by-products concentrated into approximately several %. A very small amount of diphenyl carbonate in the column bottom component ($B_B$) discharged from the column bottom is also one of the features of the present embodiment. The amount is usually 0.05 to 0.5% based on the supplied diphenyl carbonate.

Highly pure diphenyl carbonate is usually continuously discharged at a flow rate of 1 ton/hr or more, preferably 3 ton/hr or more, more preferably 5 ton/hr or more, from the side cut discharge port B2. This amount usually corresponds to approximately 90 to 96% by mass of the diphenyl carbonate supplied into the purifying column B.

The purity of the diphenyl carbonate obtained as the side cut component ($B_S$) in the step (III) is usually 99.9% or more, preferably 99.99% or more, more preferably 99.999% or more. In the present embodiment, the high purity means 99.9% or more and is preferably 99.99% or more, more preferably 99.999% or more. The contents of high-boiling impurities in high-purity diphenyl carbonate obtained when the step (II) and the step (III) are carried out with dimethyl carbonate and phenol as raw materials are 30 ppm or less, preferably 10 ppm or less, more preferably 1 ppm or less, of phenyl salicylate, 30 ppm or less, preferably 10 ppm or less, more preferably 1 ppm or less, of xanthone, 30 ppm or less, preferably 10 ppm or less, more preferably 1 ppm or less, of phenyl methoxybenzoate, and 30 ppm or less, preferably 10 ppm or less, more preferably 5 ppm or less, of 1-phenoxycarbonyl-2-phenoxycarboxy-phenylene. The total content of these high-boiling by-products is 100 ppm or less, preferably 50 ppm or less, more preferably 10 ppm or less.

In the present embodiment, the purity of the diphenyl carbonate and the content of impurities can be measured by methods described in Examples mentioned later.

In the present embodiment, halogen-free raw materials and a catalyst are usually used. Therefore, the halogen content of the resulting diphenyl carbonate is 0.1 ppm or less, preferably 10 ppb or less, more preferably 1 ppb or less (which falls outside a detection limit in ion chromatography).

In the step (III), the reflux ratio of the diphenyl carbonate purifying column B is in the range of 0.01 to 10, preferably in the range of 0.1 to 8, further preferably 0.5 to 5.

The material constituting the high-boiling substance separation column A, the diphenyl carbonate purifying column B, and a liquid-contacting part used in the present embodiment is mainly a metal material such as carbon steel or stainless steel, and stainless steel is preferred from the viewpoint of the quality of the produced diphenyl carbonate.

By carrying out the method of the present embodiment, for example, high-purity diphenyl carbonate necessary for producing high-quality and high-performance aromatic polycarbonate can be produced at an industrial scale of preferably 1 ton or more, more preferably 2 tons or more, further preferably 3 tons or more, per hour from cyclic carbonate and phenol. In the present embodiment, the industrial scale means 1 ton or more per hour and is preferably 2 tons or more, more preferably 3 tons or more. Furthermore, the halogen content of the diphenyl carbonate obtained by carrying out the method of the present embodiment is preferably 0.1 ppm or less, more preferably 10 ppb or less, further preferably 1 ppb or less, and this diphenyl carbonate contains preferably 100 ppm or less, more preferably 50 ppm or less, further preferably 10 ppm or less, of high-boiling impurities.

By carrying out the method of the present embodiment, such high-purity diphenyl carbonate can be stably produced for a long period, for example, preferably 2000 hours or longer, more preferably 3000 hours or longer, further preferably 5000 hours or longer. Thus, the method of the present embodiment is excellently effective as a method for industrially producing high-purity diphenyl carbonate.

Examples

Hereinafter, the present invention will be described further specifically with reference to Examples. However, the present invention is not limited by the following Examples.

[Analysis method]

The analysis of each composition such as the concentrations of diphenyl carbonate (DPC), a high-boiling substance (HB), and a catalyst in a column bottom component ($A_B$), the concentration of alkyl aryl carbonate in a second column low-boiling reaction mixture, and the purity or impurity component content of diphenyl carbonate was conducted by gas chromatography. The analysis by gas chromatography was conducted by the internal standard method, and the internal standard substance used was toluene.

Apparatus: 7890A manufactured by Agilent Technologies, Inc.
Column: DB-1 (Length 30 m × I.D. 0.25 mm × Film 0.25 μm) manufactured by Agilent J&W
Carrier gas: He at a flow rate of 2.0 mL/min
Injection port temperature: 250°C
Injection volume: 1.0 μL
Oven temperature: 50°C kept for 2 minutes, followed by heating at a heating rate of 10°C/min to 300°C, which was kept for 8 minutes.
Injection method: split method with a split ratio of 20 Detector: FID with a temperature of 300°C

0.1 g of a sampled column bottom component (Ab) was weighed, and 0.04 g of toluene (precisely weighed on a 0.1-mg basis in all cases) and 5 mL of acetonitrile were added thereto to prepare a sample solution for gas chromatography. The prepared solution was filtered through a 0.45 to 0.50 μm polytetrafluoroethylene (PTFE) membrane filter. The relationship between the peak area ratio and concentration ratio of DPC and the internal standard (toluene) was determined by measuring in advance each of DPC and toluene having known concentrations, and the gas chromatography measurement value of the column bottom component was corrected. A remaining amount [% by mass] determined by subtracting the DPC concentration [% by weight] obtained by gas chromatography measurement from 100 [% by mass] is the amount of the high-boiling substance (HB) and the catalyst.

In catalyst concentration measurement, organic materials of the column bottom component (Ab) were degraded with microwave. Then, the metal concentration [% by mass] of the catalyst was measured using an ICP-optical emission spectrometer (ICP). The amount of the catalyst [% by mass] was determined by calculation from the molecular weight of the catalyst.

The content of halogen in a side cut component of a diphenyl carbonate purifying column B was measured by ion chromatography.

<Example 1>

<First continuous multistage distillation column 101>

The first continuous multistage distillation column used was a continuous multistage distillation column with $L_1$ = 3300 cm, $D_1$ = 500 cm, $L_1/D_1$ = 6.6, $n_1$ = 80, $D_1/d_{11}$ = 17, and $D_1/d_{12}$ = 9 as shown in Figure 2. In this Example, the internal used was a sieve tray with cross section per hole = approximately 1.5 $cm^2$ and the number of holes = approximately 250 holes/$m^2$.

<Second continuous multistage distillation column 201>

The second continuous multistage distillation column used was a continuous multistage distillation column with $L_2$ = 3100 cm, $D_2$ = 500 cm, $L_2/D_2$ = 6.2, $n_2$ = 30, $D_2/d_{21}$ = 3.85, and $D_2/d2_2$ = 11.1 as shown in Figure 3. In this Example, the internal used was two packings of Mellapak (total theoretical number of stages: 11) installed in an upper portion and a sieve tray with cross section per hole = approximately 1.3 $cm^2$ and the number of holes = approximately 250 holes/$m^2$ in a lower portion. A material containing 2.5% by mass of a Mo component and 17% by mass of a Cr component was used in a liquid-contacting part of a lower portion. A test piece 1 containing no Mo component and containing 19% by mass of the Cr component, and a test piece 2 containing 0.15% by mass of the Mo component and 0.4% by mass of the Cr component were placed as test pieces in the liquid-contacting part of the lower portion.

<High-boiling substance separation column A>

The high-boiling substance separation column A used was a continuous multistage distillation column with $L_A$ = 1700 cm and $D_A$ = 340 cm in which Mellapak with $n_A$ = 30 was installed as the internal, as shown in Figure 5.

<Diphenyl carbonate purifying column B>

The diphenyl carbonate purifying column B used was a continuous multistage distillation column with $L_B$ = 2200 cm and $D_B$ = 280 cm in which three packings of Mellapak with $n_{B1}$ = 12, $n_{B2}$ = 18, and $n_{B3}$ = 5 were installed as the internal, as shown in Figure 5.

<Production of diphenyl carbonate>

An apparatus comprising the first continuous multistage distillation column 101 and the second continuous

multistage distillation column 201 connected with each other, as shown in Figure 4, and an apparatus comprising the high-boiling substance separation column A and the diphenyl carbonate purifying column B, as shown in Figure 5 were used to produce diphenyl carbonate.

A raw material 1 consisting of phenol/dimethyl carbonate = 1.9 (mass ratio) was continuously introduced in a liquid form at a flow rate of 57 ton/hr from an upper introduction port 11 of the first continuous multistage distillation column 101. Meanwhile, a raw material 2 consisting of dimethyl carbonate/phenol = 3.6 (mass ratio) was continuously introduced in a gas form at a flow rate of 58 ton/hr from a lower introduction port 12 of the first continuous multistage distillation column 101. The molar ratio of the raw materials introduced to the first continuous multistage distillation column 101 was dimethyl carbonate/phenol = 1.35. $Pb(OPh)_2$ was continuously introduced as a catalyst at a flow rate of 188 kg/hr from a bottom portion 11 of the high-boiling substance separation column A to the upper introduction port 11 of the first continuous multistage distillation column 101 such that the concentration was approximately 100 ppm in the liquid of the first continuous multistage distillation column 101. In the first continuous multistage distillation column 101, reactive distillation was continuously performed under conditions involving a column bottom temperature of 225°C, a column top pressure of $7 \times 10^5$ Pa, and a reflux ratio of 0. A first column low-boiling reaction mixture of the first continuous multistage distillation column 101 containing methyl alcohol, dimethyl carbonate, phenol, and the like was continuously discharged in a gas form from a column top portion 13 and discharged at a flow rate of 39 ton/hr from a discharge port 16 through a heat exchanger 14. Meanwhile, a first column high-boiling reaction mixture of the first continuous multistage distillation column 101 containing methyl phenyl carbonate, dimethyl carbonate, phenol, diphenyl carbonate, the catalyst, and the like was continuously discharged in a liquid form from an introduction port 21 through a column bottom portion 17.

Because of reaching a stable steady state 24 hours later, the first column high-boiling reaction mixture from the column bottom portion 17 of the first continuous multistage distillation column 101 was continuously supplied directly at a flow rate of 75 ton/hr from the raw material introduction port 21 installed between the Mellapak and the sieve tray of the second continuous multistage distillation column 201. The second continuous multistage distillation column 201 was allowed to contain $Pb(OPh)_2$ as a catalyst, and reactive distillation was continuously performed under conditions involving a column bottom temperature of 210°C, a column top pressure of $3 \times 10^4$ Pa, and a reflux ratio of 0.3. Stable steady operation was able to be achieved 24 hours later. A second column low-boiling reaction mixture containing 35% by mass of dimethyl carbonate, 56% by mass of phenol, and 0.5% by mass of methyl phenyl carbonate was continuously discharged from a column top portion 23 of the second continuous multistage distillation column 201, and the flow rate at a discharge port 26 was 62 ton/hr. A second column high-boiling reaction mixture containing 38% by mass of methyl phenyl carbonate and 56% by mass of diphenyl carbonate was continuously discharged from a column bottom portion 27. The second column low-boiling reaction mixture of the second continuous multistage distillation column 201 was continuously supplied into the first continuous multistage distillation column 101 from the introduction port 11 (first circulation step). In this respect, the amounts of dimethyl carbonate and phenol newly supplied were adjusted such that the composition and amounts of the raw material 1 and the raw material 2 were maintained, in light of the composition and the amount of the second column low-boiling reaction mixture.

Because of reaching a stable steady state 24 hours later, the second column high-boiling reaction mixture from the column bottom portion 27 of the second continuous multistage distillation column 201 was continuously introduced directly at 13.1 ton/hr to the introduction port A1 of the high-boiling substance separation column A. Distillation was continuously performed at a column bottom temperature ($T_A$) of 206°C, a column top pressure ($P_A$) of 1700 Pa, and a reflux ratio of 0.6 in the high-boiling substance separation column A. In this respect, the composition of the column bottom portion of the high-boiling substance separation column A was 33% by mass of diphenyl carbonate, 63% by mass of a high-boiling substance, and 4% by mass of the catalyst. In short, in the column bottom component ($A_B$) of the high-boiling substance separation column A, the mass concentration ratio (DPC/HB) of the diphenyl carbonate (DPC) to the high-boiling substance (HB) was 0.5, and the mass concentration ratio (DPC/catalyst) of the diphenyl carbonate (DPC) to the catalyst was 8.3.

A column top component ($A_T$) was continuously discharged at 12.9 ton/hr from a column top portion 13 of the high-boiling substance separation column A through a pipe 16. The column top component ($A_T$) was continuously introduced directly to the diphenyl carbonate purifying column B from an introduction port B1. A column bottom component ($A_B$) was continuously discharged at 200 kg/hr from a column bottom portion of the high-boiling substance separation column A through a pipe 11. A 12 kg/hr aliquot thereof was discharged to the outside of the system so as to keep the concentration of a formed high-boiling substance at 63% by mass. The remaining 188 kg/hr aliquot was introduced to the upper introduction port 11 of the first continuous multistage distillation column 101 (second circulation step).

Distillation was continuously performed at a column bottom temperature ($T_B$) of 213°C, a column top pressure ($P_B$) of 5000 Pa, and a reflux ratio of 1.5 in the diphenyl carbonate purifying column B. A column top component ($B_T$) was continuously discharged at 5.7 ton/hr through a pipe 26, a column bottom component ($B_B$) was continuously discharged at 30 kg/hr from a pipe 31, and a side cut component ($B_S$) was continuously discharged at 7.2 ton/hr through a pipe 33.

The composition of each component 24 hours later when the system was completely stable was as follows: the column bottom component of the first continuous multistage distillation column 101: 18.2% by mass of methyl phenyl carbonate and 2.4% by mass of diphenyl carbonate; the column top component of the high-boiling substance separation column A: 8% by mass of a low-boiling substance, 34% by mass of methyl phenyl carbonate, and 58% by mass of diphenyl carbonate from methyl phenyl carbonate; the column top component of the diphenyl carbonate purifying column B: 18% by mass of a low-boiling substance, 78% by mass of methyl phenyl carbonate, and 4% by mass of diphenyl carbonate from methyl phenyl carbonate; and the column bottom component of the diphenyl carbonate purifying column B: 95% by mass of diphenyl carbonate, 5% by mass of a high-boiling substance. All the contents of phenyl salicylate, xanthone, and phenyl methoxybenzoate in the side cut component of the diphenyl carbonate purifying column B were 1 ppm or less, and the content of 1-phenoxycarbonyl-2-phenoxycarboxy-phenylene was 4 ppm. The content of halogen in the side cut component of the diphenyl carbonate purifying column B was 1 ppb or less. This revealed that the purity of diphenyl carbonate obtained from a side cut of the diphenyl carbonate purifying column B was 99.999% or more. The production amount of this high-purity diphenyl carbonate (DPC) was 7.2 tons per hour. In this respect, the rate of conversion of dimethyl carbonate (DMC) in the first continuous multistage distillation column 101 and the rate of conversion of methyl phenyl carbonate (MPC) in the second continuous multistage distillation column 201 were 10% and 74%, respectively, in order, and the amounts of vapor used in the first continuous multistage distillation column 101 and the second continuous multistage distillation column 201 were 15.1 ton/hr and 12.4 ton/hr, respectively, in order. The amount of a makeup catalyst replenished for compensating for the catalyst discharged to the outside of the system from the column bottom portion of the high-boiling substance separation column A was 2 kg/hr.

Long-term continuous operation was performed under these conditions. The production amount and purity of diphenyl carbonate, the amounts of vapor used in the first continuous multistage distillation column 101 and the second continuous multistage distillation column 201, and the amount of a makeup catalyst replenished had substantially no change 500 hours later, 2000 hours later, 4000 hours later, 5000 hours later, and 6000 hours later. Furthermore, no corrosion was observed in a liquid-contacting part in a lower portion of the second continuous multistage distillation column 201. On the other hand, corrosion was observed in the test piece 1 and the test piece 2. Particularly, severe corrosion was observed in the test piece 2.

<Example 2>

Diphenyl carbonate was produced using the same apparatuses as in Example 1.

In this respect, the concentration of methyl phenyl carbonate in the second column low-boiling reaction mixture of the column top portion of the second continuous multistage distillation column 201 was 2.0% by mass.

As a result, the concentration of methyl phenyl carbonate in the column bottom portion of the first continuous multistage distillation column 101 was 18.2% by mass, whereas the concentration of diphenyl carbonate was 2.2% by mass. Thus, for adjusting the production amount of diphenyl carbonate to 7.2 tons per hour, it was necessary to continuously introduce the raw material 1 consisting of phenol/dimethyl carbonate = 1.9 (mass ratio) in a liquid form at a flow rate of 58 ton/hr from the upper introduction port 11 of the first continuous multistage distillation column 101, and the raw material 2 consisting of dimethyl carbonate/phenol = 3.6 (mass ratio) in a gas form at a flow rate of 60 ton/hr from the lower introduction port 12 of the first continuous multistage distillation column 101.

As a result, the rate of conversion of dimethyl carbonate (DMC) in the first continuous multistage distillation column 101 and the rate of conversion of methyl phenyl carbonate (MFC) in the second continuous multistage distillation column 201 were 9.8% and 72%, respectively, in order, and the amounts of vapor used in the first continuous multistage distillation column 101 and the second continuous multistage distillation column 201 were 15.4 ton/hr and 12.7 ton/hr, respectively, in order.

<Comparative Example 1>

Diphenyl carbonate was produced using the same apparatuses as in Example 1.

In this respect, the composition of the column bottom portion of the high-boiling substance separation column A was 58% by mass of diphenyl carbonate, 38% by mass of a high-boiling substance, and 4% by mass of the catalytic component. In short, in the column bottom component ($A_B$) of the high-boiling substance separation column A, the mass concentration ratio (DPC/HB) of the diphenyl carbonate (DPC) to the high-boiling substance (HB) was 1.5, and the mass concentration ratio (DPC/catalyst) of the diphenyl carbonate (DPC) to the catalyst was 14.5.

As a result, the concentration of methyl phenyl carbonate in the column bottom portion of the first continuous multistage distillation column 101 was 18.2% by mass, whereas the concentration of diphenyl carbonate was 0.8% by mass. Thus, for adjusting the production amount of diphenyl carbonate to 7.2 tons per hour, it was necessary to continuously introduce the raw material 1 consisting of phenol/dimethyl carbonate = 1.9 (mass ratio) in a liquid form at a flow rate of 63 ton/hr from the upper introduction port 11 of the first continuous multistage distillation column 101, and the

raw material 2 consisting of dimethyl carbonate/phenol = 3.6 (mass ratio) in a gas form at a flow rate of 64 ton/hr from the lower introduction port 12 of the first continuous multistage distillation column 101. For maintaining the concentration of the high-boiling substance at 38% by mass, it was also necessary to discharge the column bottom component at a flow rate of 20 kg/hr to the outside of the system from the column bottom portion of the high-boiling substance separation column A.

As a result, the rate of conversion of dimethyl carbonate (DMC) in the first continuous multistage distillation column 101 and the rate of conversion of methyl phenyl carbonate (MFC) in the second continuous multistage distillation column 201 were both deteriorated by 10% as compared with the case of Example 1, and the amounts of vapor used in the first continuous multistage distillation column 101 and the second continuous multistage distillation column 201 were also increased by 9% and 10%, respectively, in order, as compared with the case of Example 1. The amount of a makeup catalyst replenished for compensating for the catalyst discharged to the outside of the system from the column bottom portion of the high-boiling substance separation column A was also 1.5 times the amount in Example 1.

<Comparative Example 2>

Diphenyl carbonate was produced using the same apparatuses as in Example 1.

In this respect, the composition of the column bottom portion of the high-boiling substance separation column A was 56% by mass of diphenyl carbonate, 37% by mass of a high-boiling substance, and 7% by mass of the catalytic component. In short, in the column bottom component ($A_B$) of the high-boiling substance separation column A, the mass concentration ratio (DPC/HB) of the diphenyl carbonate (DPC) to the high-boiling substance (HB) was 1.5, and the mass concentration ratio (DPC/catalyst) of the diphenyl carbonate (DPC) to the catalyst was 8.3.

As a result, for maintaining the concentration of the high-boiling substance at 37% by mass, it was necessary to discharge the column bottom component ($A_B$) at a flow rate of 20 kg/hr to the outside of the system from the column bottom portion of the high-boiling substance separation column A. As a result, the amount of a makeup catalyst replenished for compensating for the catalyst discharged to the outside of the system from the column bottom portion of the high-boiling substance separation column A was also 3.5 times the amount in Example 1.

<Comparative Example 3>

Diphenyl carbonate was produced using the same apparatuses as in Example 1.

In this respect, the composition of the column bottom portion of the high-boiling substance separation column A was 35% by mass of diphenyl carbonate, 63% by mass of a high-boiling substance, and 2% by mass of the catalytic component. In short, in the column bottom component ($A_B$) of the high-boiling substance separation column A, the mass concentration ratio (DPC/HB) of the diphenyl carbonate (DPC) to the high-boiling substance (HB) was 0.5, and the mass concentration ratio (DPC/catalyst) of the diphenyl carbonate (DPC) to the catalyst was 14.5.

As a result, the concentration of methyl phenyl carbonate in the column bottom portion of the first continuous multistage distillation column 101 was 18.2% by mass, whereas the concentration of diphenyl carbonate was 0.8% by mass. Thus, for adjusting the production amount of diphenyl carbonate to 7.2 tons per hour, it was necessary to continuously introduce the raw material 1 consisting of phenol/dimethyl carbonate = 1.9 (mass ratio) in a liquid form at a flow rate of 63 ton/hr from the upper introduction port 11 of the first continuous multistage distillation column 101, and the raw material 2 consisting of dimethyl carbonate/phenol = 3.6 (mass ratio) in a gas form at a flow rate of 64 ton/hr from the lower introduction port 12 of the first continuous multistage distillation column 101.

As a result, the rate of conversion of dimethyl carbonate (DMC) in the first continuous multistage distillation column 101 and the rate of conversion of methyl phenyl carbonate (MPC) in the second continuous multistage distillation column 201 were both deteriorated by 10% as compared with the case of Example 1, and the amounts of vapor used in the first continuous multistage distillation column 101 and the second continuous multistage distillation column 201 were also increased by 9% and 10%, respectively, in order, as compared with the case of Example 1.

<Example 3>

Diphenyl carbonate was produced by use of the same apparatuses and production method as in Example 1 except that the catalyst was changed to a catalyst described in Example 1 of International Publication No. WO 2011/105442.

(Preparation of titanium-containing composition)

A 60 L batch reactor equipped with a stirrer, a heater, and a distillation column was charged with 7 kg of tetrabutoxytitanium (manufactured by DuPont de Nemours, Inc., product name: Tyzor TnBT) in a nitrogen atmosphere

and subsequently charged with 14 kg of phenol purified by distillation in advance.

Next, the mixture in the batch reactor was heated to 180°C by the heater at ordinary temperature and reacted. n-Butanol generated through the reaction was recovered from the column top of the distillation column. Further, the pressure of the batch reactor was reduced to approximately 53 kPa, and n-butanol was recovered.

Then, the batch reactor was returned to ordinary pressure and charged with approximately 18 kg of diphenyl carbonate, and the mixture in the reactor was heated to approximately 190°C. Next, the pressure of the batch reactor was reduced to approximately 1.3 kPa, and diphenyl carbonate containing a low-boiling component was distilled off to obtain a titanium-containing composition. Diphenyl carbonate was added to the obtained titanium-containing composition such that its titanium concentration was 5% by mass. The titanium-containing composition was heated at 200°C for approximately 48 hr and then used as a reaction catalyst in the production of diphenyl carbonate.

The titanium-containing composition catalyst was continuously introduced as a catalyst at a flow rate of 188 kg/hr from the bottom portion 11 of the high-boiling substance separation column A to the upper introduction port 11 of the first continuous multistage distillation column 101 such that the concentration was approximately 100 ppm in the liquid of the first continuous multistage distillation column 101.

<Example 4>

Diphenyl carbonate was produced by use of the same apparatuses and production method as in Example 2 except that the catalyst was changed to the same catalyst as in Example 3.

Table 1 shows respective conditions and results of Examples 1 to 4 and Comparative Examples 1 to 3.

[Table 1]

| | | | Example 1 | Example 2 | Example 3 | Example 4 | Comparative Example 1 | Comparative Example 2 | Comparative Example 3 |
|---|---|---|---|---|---|---|---|---|---|
| Conditions | Column bottom component ($A_B$) of high-boiling substance separation column A | DPC/HB | 0.5 | 0.5 | 0.5 | 0.5 | 1.5 | 1.5 | 0.5 |
| | | DPC/catalyst | 8.3 | 8.3 | 8.3 | 8.3 | 14.5 | 8.3 | 14.5 |
| | | DPC (% by mass) | 33 | 33 | 33 | 33 | 58 | 56 | 35 |
| | | HB (% by mass) | 63 | 63 | 63 | 63 | 38 | 37 | 63 |
| | | Catalyst (% by mass) | 4 | 4 | 4 | 4 | 4 | 6.8 | 2 |
| | MPC concentration in second column low-boiling reaction mixture (% bv mass) | | 0.5 | 2 | 0.5 | 2 | 0.5 | 0.5 | 0.5 |

(continued)

| | | | Example 1 | Example 2 | Example 3 | Example 4 | Comparative Example 1 | Comparative Example 2 | Comparative Example 3 |
|---|---|---|---|---|---|---|---|---|---|
| Results | Yield | DPC (ton/hr) | 7.2 | 7.2 | 7.2 | 7.2 | 7.2 | 7.2 | 7.2 |
| | Rate of conversion | DMC (%) of first continuous multistage distillation column 101 | 10 | 9.8 | 10 | 98 | 9 | 10 | 9 |
| | | MPC (%) of first continuous multistage distillation column 101 | 36 | 35 | 36 | 35 | 33 | 36 | 33 |
| | | MPC (%) of second continuous multistage distillation column 201 | 74 | 72 | 74 | 72 | 67 | 74 | 67 |
| | Selectivity | MPC (%) of first continuous multistage distillation column 101 | 98 | 98 | 98 | 98 | 98 | 98 | 98 |
| | | DPC (%) of second continuous multistage distillation column 201 | 99 | 99 | 99 | 99 | 99 | 99 | 99 |
| | Amount of circulation | Raw material (recycled phenol) (ton/hr) | 57 | 58 | 57 | 58 | 63 | 57 | 63 |
| | | Raw material (vapor) (ton/hr) | 58 | 60 | 58 | 60 | 64 | 58 | 64 |
| | Amount of vapor used | First continuous multistage distillation column 101 (ton/hr) | 15.1 | 15.4 | 15.0 | 15.3 | 16.4 | 15.1 | 16.4 |
| | | Second continuous multistage distillation column 201 (ton/hr) | 12.4 | 12.7 | 12.3 | 12.6 | 13.7 | 12.4 | 13.7 |
| | Amount of makeup catalyst replenished (kg/hr) | | 2 | 2 | 2 | 2 | 3 | 7 | 1 |

The present application is based on Japanese Patent Application No. 2021-172658 filed on October 21, 2021, the contents of which are incorporated herein by reference.

Industrial Applicability

By carrying out the method of the present invention, for example, high-purity diphenyl carbonate necessary for producing high-quality and high-performance aromatic polycarbonate can be produced at an industrial scale of 1 ton or more, preferably 2 tons or more, more preferably 3 tons or more, per hour in a state where a heat usage can be reduced and the amount of a catalyst replenished can be reduced. Furthermore, high-purity diphenyl carbonate having a halogen content of 0.1 ppm or less, preferably 10 ppb or less, more preferably 1 ppb or less, and containing 100 ppm or less, preferably 50 ppm or less, more preferably 10 ppm or less, of high-boiling impurities can be stably produced for a long period, for example, 2000 hours or longer, preferably 3000 hours or longer, more preferably 5000 hours or longer. Thus, the method of the present invention is excellently effective as a method for industrially producing high-purity diphenyl carbonate.

Reference Signs List

(Figure 1) 1: gas discharge port, 2: liquid discharge port, 3-a to 3-e: introduction port, 4-a to 4-b: introduction port, 5: head plate part, 6: internal, 7: body part, 10: continuous multistage distillation column, $L_0$: body length (cm), $D_0$: body inside diameter (cm), $d_{01}$: inside diameter (cm) of gas discharge port, $d_{02}$: inside diameter (cm) of liquid discharge port

(Figures 2, 3, and 4) 1: gas discharge port, 2: liquid discharge port, 3: introduction port, 4: introduction port, 5: head plate part, $L_1$ and $L_2$: body length (cm), $D_1$ and $D_2$: body inside diameter (cm), $d_{11}$ and $d_{21}$: gas discharge port inside diameter (cm), $d_{12}$ and $d_{22}$: liquid discharge port inside diameter (cm), 101: first continuous multistage distillation column, 201: second continuous multistage distillation column, 11, 12, and 21: introduction port, 13 and 23: column top gas discharge port, 14, 24, 18, and 28: heat exchanger, 17 and 27: column bottom liquid discharge port, 16, 26: column top component discharge port, 31: column bottom component discharge port of second continuous multistage distillation column

(Figure 5) A1 and B1: introduction port, B2: discharge port, 11: column bottom component discharge port of high-boiling substance separation column A, 13 and 23: column top gas discharge port, 14, 24, 18, and 28, : heat exchanger, 15 and 25: reflux liquid introduction port, 16: column top component discharge port of high-boiling substance separation column A, 17 and 27: column bottom liquid discharge port, 26: column top component discharge port of diphenyl carbonate purifying column B, 31: column bottom component discharge port of diphenyl carbonate purifying column B, 33: side cut component discharge port of diphenyl carbonate purifying column B

## Claims

**1.** A method for continuously producing diphenyl carbonate, comprising:

the step of continuously supplying dialkyl carbonate and phenol into a first continuous multistage distillation column containing a catalyst, simultaneously performing a reaction and distillation in the first continuous multistage distillation column, continuously discharging a first column low-boiling reaction mixture containing alcohols thus formed in a gas form from an upper portion of the first continuous multistage distillation column, and continuously discharging a first column high-boiling reaction mixture containing alkyl phenyl carbonate thus formed in a liquid form from a lower portion of the first continuous multistage distillation column to continuously produce alkyl phenyl carbonate;

the step of continuously supplying the first column high-boiling reaction mixture into a second continuous multistage distillation column containing a catalyst, simultaneously performing a reaction and distillation in the second continuous multistage distillation column, continuously discharging a second column low-boiling reaction mixture containing dialkyl carbonates thus formed in a gas form from an upper portion of the second continuous multistage distillation column, and continuously discharging a second column high-boiling reaction mixture containing diphenyl carbonate thus formed in a liquid form from a lower portion of the second continuous multistage distillation column to continuously produce diphenyl carbonate;

a first purification step of continuously introducing the second column high-boiling reaction mixture containing diphenyl carbonate to a high-boiling substance separation column A, and continuously separating by distillation

the mixture into a column top component ($A_T$) containing diphenyl carbonate and a column bottom component ($A_B$) containing the catalyst;

a second purification step of continuously introducing the column top component ($A_T$) to a diphenyl carbonate purifying column B having a side cut discharge port, and continuously separating by distillation the component into three components, a column top component ($B_T$), a side cut component ($B_S$), and a column bottom component ($B_B$), thereby obtaining diphenyl carbonate as the side cut component ($B_S$);

a first circulation step of continuously supplying the second column low-boiling reaction mixture into the first continuous multistage distillation column; and

a second circulation step of continuously supplying a portion of the column bottom component ($A_B$) into the first continuous multistage distillation column and/or into the second continuous multistage distillation column, wherein

the column bottom component ($A_B$) of the high-boiling substance separation column A contains diphenyl carbonate (DPC) and a high-boiling substance (HB), wherein a mass concentration ratio (DPC/HB) of the diphenyl carbonate to the high-boiling substance in the column bottom component ($A_B$) is 0.1 to 1.0, and a mass concentration ratio (DPC/catalyst) of the diphenyl carbonate to the catalyst is 1.0 to 10.0.

**2.** The production method according to claim 1,
wherein a concentration of the diphenyl carbonate in the column bottom component ($A_B$) is 9 to 38% by mass.

**3.** The production method according to claim 1 or 2,
wherein a material that is composed mainly of Fe and contains 2% by mass or more of Mo and 18% by mass or less of Cr is used in a liquid-contacting part in a lower portion of the second continuous multistage distillation column.

**4.** The production method according to claim 1 or 2,
wherein a concentration of alkyl aryl carbonate in the second column low-boiling reaction mixture is 1% by mass or less.

**5.** The production method according to claim 1 or 2, wherein

(a) the first continuous multistage distillation column is a continuous multistage distillation column that comprises a cylindrical body with a length $L_1$ (cm) and an inside diameter $D_1$ (cm), comprises a structure having an internal having the number of stages $n_1$ in the inside, and comprises a gas discharge port with an inside diameter $d_{11}$ (cm) in a column top portion or an upper portion of the column close thereto, a liquid discharge port with an inside diameter $d_{12}$ (cm) in a column bottom portion or a lower portion of the column close thereto, one or more third introduction ports in an upper portion and/or an intermediate portion of the column below the gas discharge port, and one or more fourth introduction ports in an intermediate portion and/or a lower portion of the column above the liquid discharge port, wherein $L_1$, $D_1$, $L_1/D_1$, $n_1$, $D_1/d_{11}$, and $D_1/d_{12}$ respectively satisfy the following expressions (7) to (12):

$$1500 \leq L_1 \leq 8000 \text{ ... Expression (7)}$$

$$100 \leq D_1 \leq 2000 \text{ ... Expression (8)}$$

$$2 \leq L_1/D_1 \leq 40 \text{ ... Expression (9)}$$

$$20 \leq n_1 \leq 120 \text{ ... Expression (10)}$$

$$5 \leq D_1/d_{11} \leq 30 \text{ ... Expression (11)}$$

$$3 \leq D_1/d_{12} \leq 20 \text{ ... Expression (12);}$$

(b) the second continuous multistage distillation column is a continuous multistage distillation column that comprises a cylindrical body with a length $L_2$ (cm) and an inside diameter $D_2$ (cm), comprises a structure having an internal having the number of stages $n_2$ in the inside, and comprises a gas discharge port with an inside diameter $d_{21}$ (cm) in a column top portion or an upper portion of the column close thereto, a liquid discharge port with an inside diameter $d_{22}$ (cm) in a column bottom portion or a lower portion of the column close thereto, one or more fifth introduction ports in an upper portion and/or an intermediate portion of the column below the gas discharge port, and one or more sixth introduction ports in an intermediate portion and/or a lower portion of the column above the liquid discharge port, wherein $L_2$, $D_2$, $L_2/D_2$, $n_2$, $D_2/d_{21}$, and $D_2/d_{22}$ respectively satisfy the following expressions (13) to (18):

$$1500 \leq L_2 \leq 8000 \text{ ... Expression (13)}$$

$$100 \leq D_2 \leq 2000 \text{ ... Expression (14)}$$

$$2 \leq L_2/D_2 \leq 40 \text{ ... Expression (15)}$$

$$10 \leq n_2 \leq 80 \text{ ... Expression (16)}$$

$$2 \leq D_2/d_{21} \leq 15 \text{ ... Expression (17)}$$

$$5 \leq D_2/d_{22} \leq 30 \text{ ... Expression (18);}$$

(c) the high-boiling substance separation column A is a continuous multistage distillation column that comprises a cylindrical body with a length $L_A$ (cm) and an inside diameter $D_A$ (cm) and comprises an internal having the number of stages $n_A$ in the inside, wherein $L_A$, $D_A$, and $n_A$ respectively satisfy the following expressions (19) to (21) :

$$800 \leq L_A \leq 3000 \text{ ... Expression (19)}$$

$$100 \leq D_A \leq 1000 \text{ ... Expression (20)}$$

$$20 \leq n_A \leq 100 \text{ ... Expression (21);}$$

and

(d) the diphenyl carbonate purifying column B is a continuous multistage distillation column that comprises a cylindrical body with a length $L_B$ (cm) and an inside diameter $D_B$ (cm), comprises a structure having an internal in the inside, and comprises an introduction port $B_1$ in an intermediate portion of the column and a side cut discharge port $B_2$ between the introduction port $B_1$ and the column bottom, wherein the number of stages of the internal from the introduction port $B_1$ through an upper portion is defined as $n_{B1}$, the number of stages of the internal between the introduction port $B_1$ and the side cut discharge port $B_2$ is defined as $n_{B2}$, the number of stages of the internal from the side cut discharge port $B_2$ through a lower portion is defined as $n_{B3}$, and the total number of stages ($n_{B1}$ + $n_{B2}$ + $n_{B3}$) is defined as $n_B$, wherein $L_B$, $D_B$, $n_{B1}$, $n_{B2}$, $n_{B3}$, and $n_B$ respectively satisfy the following expressions (22) to (27):

$$1000 \leq L_B \leq 5000 \text{ ... Expression (22)}$$

$$100 \leq D_B \leq 1000 \text{ ... Expression (23)}$$

$$5 \leq n_{B1} \leq 20 \ \ldots \ \text{Expression (24)}$$

$$12 \leq n_{B2} \leq 40 \ \ldots \ \text{Expression (25)}$$

$$3 \leq n_{B3} \leq 15 \ \ldots \ \text{Expression (26)}$$

$$20 \leq n_{B} \leq 70 \ \ldots \ \text{Expression (27)}$$

[Figure 1]

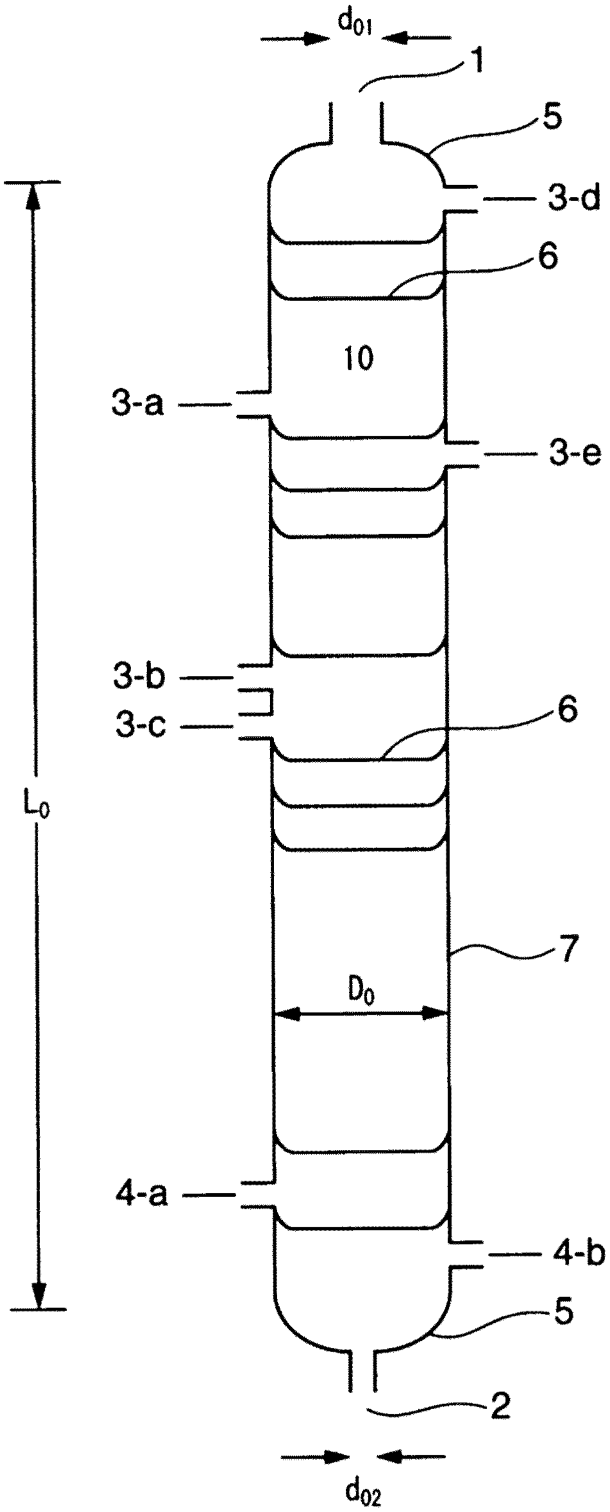

[Figure 2]

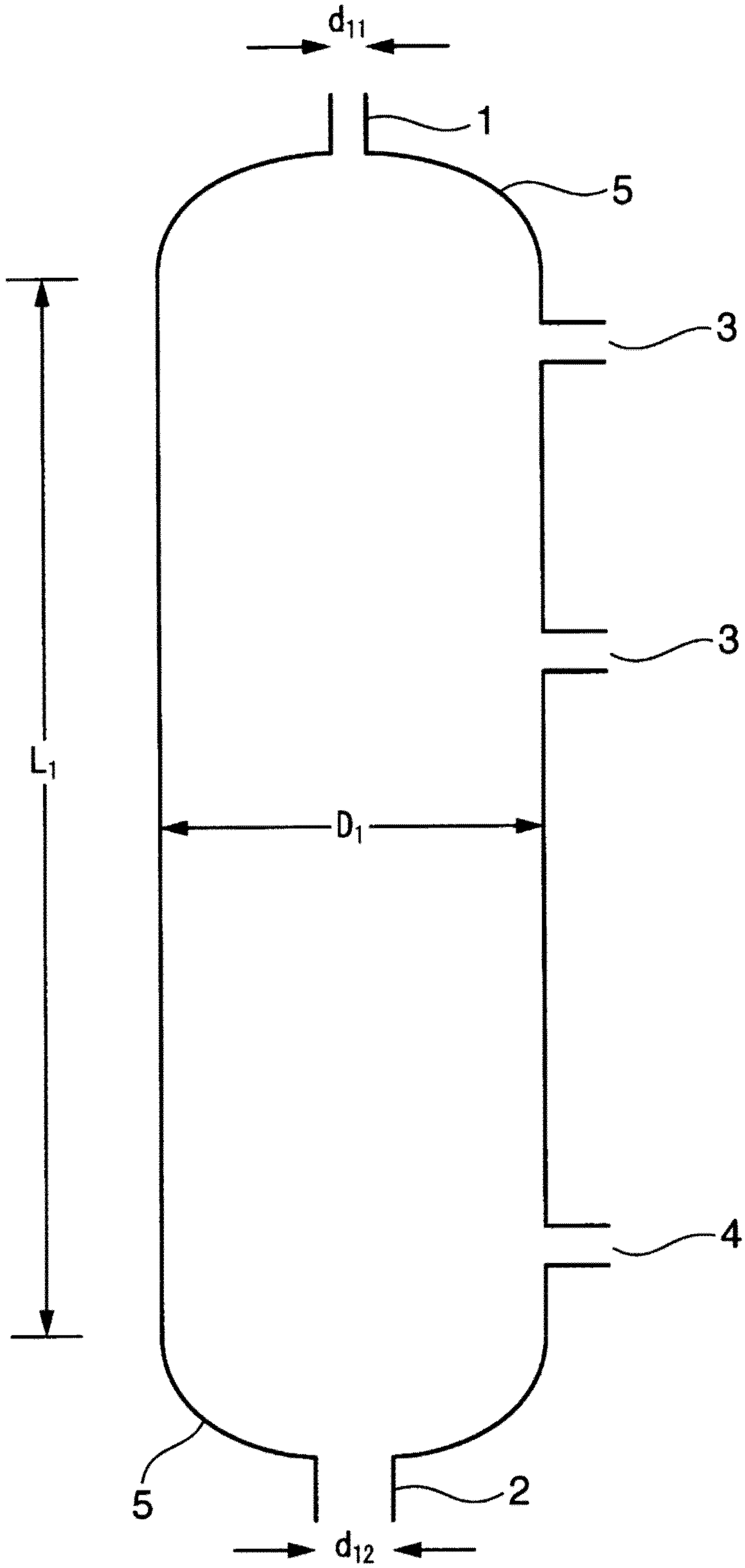

[Figure 3]

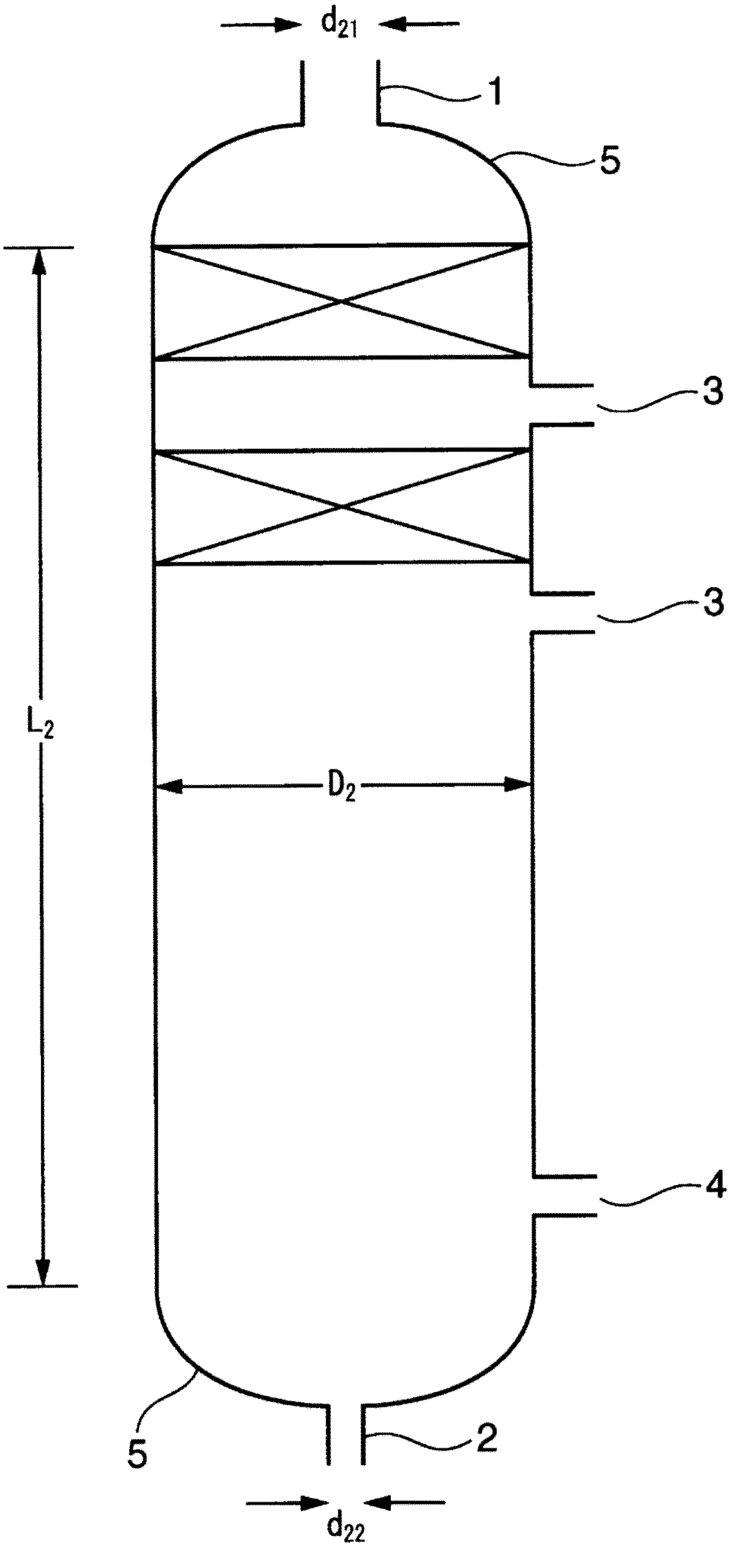

[Figure 4]

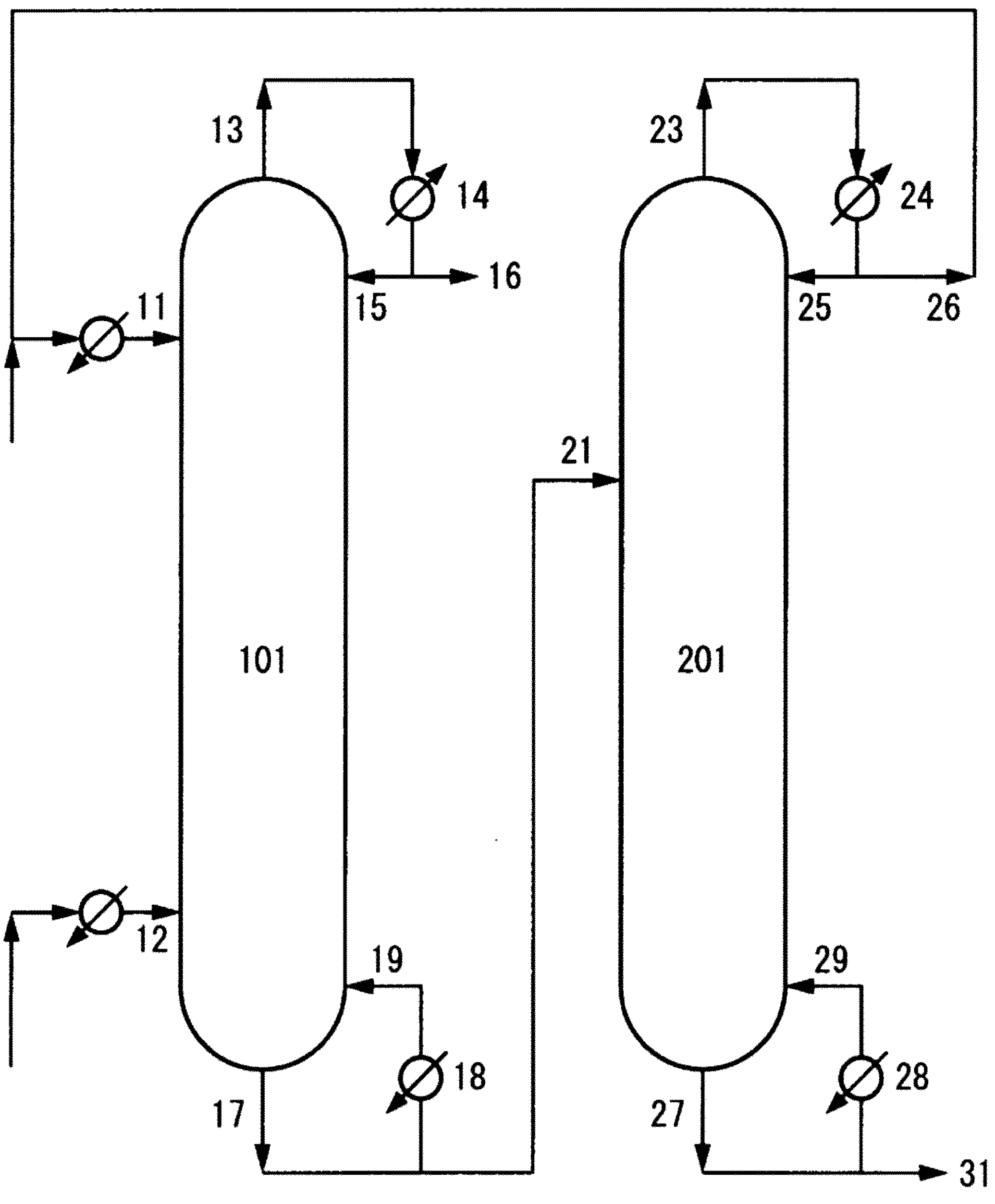

[Figure 5]

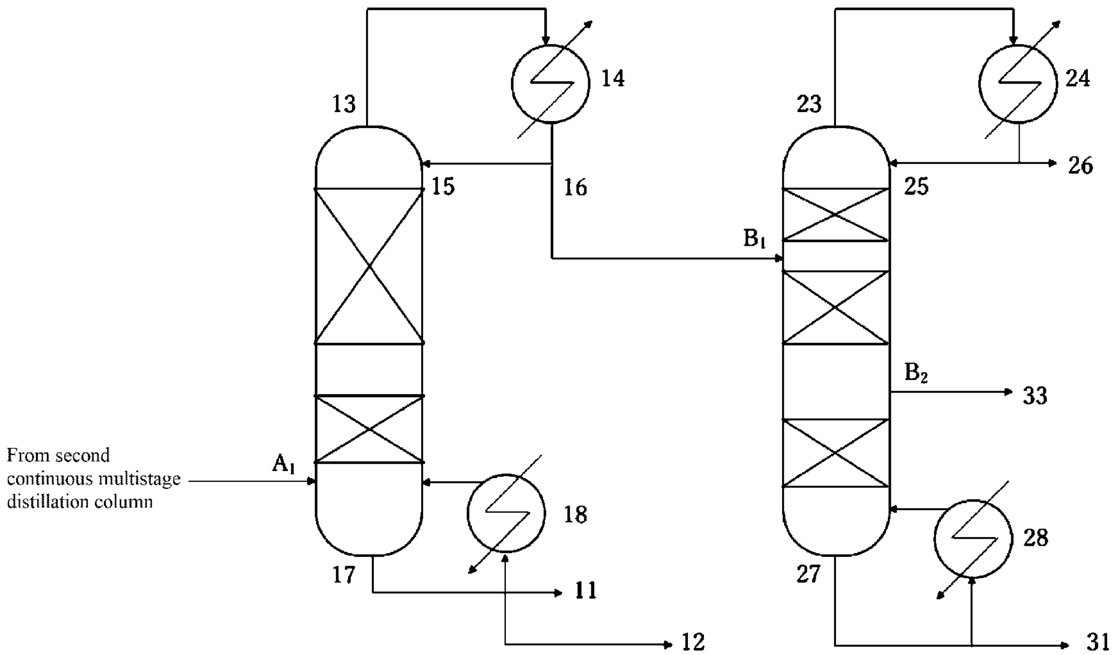
13
14
15
16
17
18
11
12
A1
From second continuous multistage distillation column
23
24
25
26
B1
B2
33
27
28
31

## INTERNATIONAL SEARCH REPORT

International application No.
**PCT/JP2022/038887**

**A. CLASSIFICATION OF SUBJECT MATTER**

***C07C 68/06***(2020.01)i; ***C07C 68/08***(2006.01)i; ***C07C 69/96***(2006.01)i
FI: C07C68/08; C07C69/96 Z; C07C68/06

According to International Patent Classification (IPC) or to both national classification and IPC

**B. FIELDS SEARCHED**

Minimum documentation searched (classification system followed by classification symbols)

C07C68/06; C07C68/08; C07C69/96

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2011/105442 A1 (ASAHI KASEI CHEMICALS CORP.) 01 September 2011 (2011-09-01)<br>claim 1, comparative example 14, table 6, fig. 1 | 1-2<br>3-5 |
| A | WO 2011/105439 A1 (ASAHI KASEI CHEMICALS CORP.) 01 September 2011 (2011-09-01)<br>entire text, all drawings | 1-5 |
| A | WO 2007/069463 A1 (ASAHI KASEI CHEMICALS CORP.) 21 June 2007 (2007-06-21)<br>entire text, all drawings | 1-5 |
| A | WO 2006/041075 A1 (ASAHI KASEI CHEMICALS CORP.) 20 April 2006 (2006-04-20)<br>entire text, all drawings | 1-5 |
| A | JP 11-092429 A (ASAHI CHEM. IND. CO., LTD.) 06 April 1999 (1999-04-06)<br>entire text, all drawings | 1-5 |
| A | JP 11-049727 A (ASAHI CHEM. IND. CO., LTD.) 23 February 1999 (1999-02-23)<br>entire text, all drawings | 1-5 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 December 2022** | **27 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**

International application No.
**PCT/JP2022/038887**

**C. DOCUMENTS CONSIDERED TO BE RELEVANT**

| Category* | Citation of document, with indication, where appropriate, of the relevant passages | Relevant to claim No. |
|---|---|---|
| A | JP 09-255772 A (ASAHI CHEM. IND. CO., LTD.) 30 September 1997 (1997-09-30) entire text, all drawings | 1-5 |
| A | WO 2006/022294 A1 (ASAHI KASEI CHEMICALS CORP.) 02 March 2006 (2006-03-02) entire text, all drawings | 1-5 |
| A | WO 2006/025424 A1 (ASAHI KASEI CHEMICALS CORP.) 09 March 2006 (2006-03-09) entire text, all drawings | 1-5 |

Form PCT/ISA/210 (second sheet) (January 2015)

**INTERNATIONAL SEARCH REPORT**
**Information on patent family members**

International application No.
**PCT/JP2022/038887**

| Patent document cited in search report | Publication date (day/month/year) | Patent family member(s) | Publication date (day/month/year) |
|---|---|---|---|
| WO 2011/105442 A1 | 01 September 2011 | US 2012/0316357 A1<br>claim 1, comparative example 14, table 6, fig. 1<br>EP 2540697 A1<br>CN 102753516 A<br>KR 10-2012-0103745 A | |
| WO 2011/105439 A1 | 01 September 2011 | US 2012/0316355 A1<br>entire text, all drawings<br>EP 2540694 A1<br>CN 102753508 A<br>KR 10-2012-0107009 A | |
| WO 2007/069463 A1 | 21 June 2007 | US 2009/0209724 A1<br>entire text, all drawings<br>EP 1961781 A1<br>CN 101331168 A<br>KR 10-2008-0067380 A | |
| WO 2006/041075 A1 | 20 April 2006 | US 2007/0270604 A1<br>entire text, all drawings<br>EP 1801095 A1<br>CN 101039896 A<br>KR 10-2008-0104196 A | |
| JP 11-092429 A | 06 April 1999 | US 6262210 B1<br>entire text, all drawings<br>EP 1016648 A1<br>CN 1265641 A<br>KR 10-2001-0022248 A | |
| JP 11-049727 A | 23 February 1999 | (Family: none) | |
| JP 09-255772 A | 30 September 1997 | US 5747609 A<br>entire text, all drawings<br>SG 52906 A<br>TW 442516 B | |
| WO 2006/022294 A1 | 02 March 2006 | US 2008/0041712 A1<br>entire text, all drawings<br>EP 1783112 A1<br>CN 101006045 A<br>KR 10-2007-0039962 A | |
| WO 2006/025424 A1 | 09 March 2006 | US 2007/0260083 A1<br>entire text, all drawings<br>EP 1787977 A1<br>CN 101010284 A<br>KR 10-2007-0039975 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007072705 A
- WO 2011105442 A
- JP 2021172658 A